# EUROPEAN PATENT SPECIFICATION

(11) **EP 1 352 068 B1**
(45) Date of publication and mention of the grant of the patent: **24.09.2008**
(21) Application number: 02722023.5
(22) Date of filing: 08.01.2002
(51) Int. Cl.: C12N 15/32, C07K 14/325, C12N 15/62, C12N 15/63, C12N 1/21, A01H 5/00

(54) **BACILLUS THURINGIENSIS INSECTICIDAL PROTEINS**
INSEKTIZIDPROTEINE AUS BACILLUS THURINGIENSIS
NOUVELLES PROTEINES INSECTICIDES DU BACILLUS THURINGIENSIS

(30) Priority: 09.01.2001 US 756296
(43) Date of publication of application: 15.10.2003
(60) Divisional application: 08075614.1
(73) Proprietor: Bayer BioScience N.V., 9052 Gent (BE)
(72) Inventor: ARNAUT, Greta, B-9910 Knesselare (BE); BOETS, Annemie, B-9620 Velzeke (BE); VANNESTE, Stijn, B-8500 Kortrijk (BE); VAN RIE, Jeroen, B-9900 Eeklo (BE); VAN HOUDT, Sara, B-9620 Zottegem (BE)
(86) International application number: PCT/EP2002/000298
(87) International publication number: WO 2002/057664

(56) References cited:
- WO-A-00/26371
- WO-A-01/19859
- WO-A-98/40490
- WIDNER W R ET AL: "TWO HIGHLY RELATED INSECTICIDAL CRYSTAL PROTEINS OF BACILLUS THURINGIENSIS SUBSP. KURSTAKI POSSESS DIFFERENT HOST RANGE SPECIFICITIES" JOURNAL OF BACTERIOLOGY, WASHINGTON, DC, US, vol. 171, no. 2, 1 February 1989 (1989-02-01), pages 965-974, XP002071367 ISSN: 0021-9193
- WU D ET AL: "SEQUENCE OF AN OPERON CONTAINING A NOVEL DELTA-ENDOTOXIN GENE FROM BACILLUS THURINGIENSIS" FEMS MICROBIOLOGY LETTERS, AMSTERDAM, NL, vol. 81, no. 1, 1 June 1991 (1991-06-01), pages 31-35, XP002071368 ISSN: 0378-1097
- KOTA M ET AL: "Overexpression of the Bacillus thuringiensis (Bt) Cry2Aa2 protein in chloroplasts confers resistance to plants against susceptible and Bt-resistant insects" PROCEEDINGS OF THE NATIONAL ACADEMY OF SCIENCES OF USA, NATIONAL ACADEMY OF SCIENCE. WASHINGTON, US, vol. 96, no. 5, 2 March 1999 (1999-03-02), pages 1840-1845, XP002132142 ISSN: 0027-8424
- SHAHINA BANO MAQBOOL ET AL: "EFFECTIVE CONTROL OF YELLOW STEM BORER AND RICE LEAF FOLDER IN TRANSGENIC RICE INDICA VARIETIES BASMATI 370 AND M 7 USING THE NOVEL DELTA-ENDOTOXIN CRY2A BACILLUS THURINGIENSIS GENE" MOLECULAR BREEDING, KLUWER ACADEMIC PUBLISHERS, DORDRECHT, NL, vol. 4, no. 6, December 1998 (1998-12), pages 501-507, XP000881596 ISSN: 1380-3743

### SEQUENCE LISTING

<110> Bayer Bioscience N.V.
<120> Novel Bacillus thuringiensis insecticidal proteins
<130> NEW2AS EPw1
<150> US 09/756296
   <151> 2001-01-09
<160> 5
<170> Patent In version 3.0
<210> 1
   <211> 1899
   <212> DNA
   <213> Bacillus thuringiensins
<220>
   <221> CDS
   <222> (1)..(1896)
<400> 1
   atg aat aat gta tta aat aac gga aga act act att tgt gat gcg tat      48
   Met Asn Asn Val Leu Asn Asn Gly Arg Thr Thr Ile Cys Asp Ala Tyr
   1               5                   10                  15
   aat gta gtg gcc cat gat cca ttt agt ttt gag cat aaa tca tta gat      96
   Asn Val Val Ala His Asp Pro Phe Ser Phe Glu His Lys Ser Leu Asp
               20                  25                  30
   acc atc cga aaa gaa tgg atg gag tgg aaa aga aca gat cat agt tta     144
   Thr Ile Arg Lys Glu Trp Met Glu Trp Lys Arg Thr Asp His Ser Leu
           35                  40                  45
   tat gta gct cct ata gtc gga act gtt tct agc ttt ctg cta aag aag     192
   Tyr Val Ala Pro Ile Val Gly Thr Val Ser Ser Phe Leu Leu Lys Lys
       50                  55                  60
   gtg ggg agt ctt att gga aaa agg ata ttg agt gaa tta tgg ggg tta     240
   Val Gly Ser Leu Ile Gly Lys Arg Ile Leu Ser Glu Leu Trp Gly Leu
   65                  70                  75                  80
   ata ttt cct agt ggt agc aca aat cta atg caa gat att tta agg gag     288
   Ile Phe Pro Ser Gly Ser Thr Asn Leu Met Gln Asp Ile Leu Arg Glu
                   85                  90                  95
   aca gaa caa ttc cta aat caa aga ctt aat aca gac act ctt gcc cgt     336
   Thr Glu Gln Phe Leu Asn Gln Arg Leu Asn Thr Asp Thr Leu Ala Arg
               100                 105                 110
   gta aat gcg gaa ttg gaa ggg ctg caa gcg aat ata agg gag ttt aat     384
   Val Asn Ala Glu Leu Glu Gly Leu Gln Ala Asn Ile Arg Glu Phe Asn
           115                 120                 125
   caa caa gta gat aat ttt tta aat cct act caa aac cct gtt cct tta      432
   Gln Gln Val Asp Asn Phe Leu Asn Pro Thr Gln Asn Pro Val Pro Leu
       130                 135                 140
   tca ata act tct tca gtt aat aca atg cag caa tta ttt cta aat aga      480
   Ser Ile Thr Ser Ser Val Asn Thr Met Gln Gln Leu Phe Leu Asn Arg
   145                 150                 155                 160
   tta ccc cag ttc cgt gtg caa gga tac caa ctg tta tta tta cct tta      528
   Leu Pro Gln Phe Arg Val Gln Gly Tyr Gln Leu Leu Leu Leu Pro Leu
                   165                 170                 175
   ttt gca cag gca gcc aat atg cat ctt tct ttt att aga gat gtt gtt      576
   Phe Ala Gln Ala Ala Asn Met His Leu Ser Phe Ile Arg Asp Val Val
               180                 185                 190
   ctc aat gca gat gaa tgg gga att tca gca gca aca tta cgt acg tat      624
   Leu Asn Ala Asp Glu Trp Gly Ile Ser Ala Ala Thr Leu Arg Thr Tyr
           195                 200                 205
   caa aat tat ctg aaa aat tat aca aca gag tac tct aat tat tgt ata      672
   Gln Asn Tyr Leu Lys Asn Tyr Thr Thr Glu Tyr Ser Asn Tyr Cys Ile
       210                 215                 220
   aat acg tat caa act gcg ttt aga ggt tta aac acc cgt tta cac gat      720
   Asn Thr Tyr Gln Thr Ala Phe Arg Gly Leu Asn Thr Arg Leu His Asp
   225                 230                 235                 240
   atg tta gaa ttt aga aca tat atg ttt tta aat gta ttt gaa tat gta      768
   Met Leu Glu Phe Arg Thr Tyr Met Phe Leu Asn Val Phe Glu Tyr Val
                   245                 250                 255
   tct atc tgg tcg ttg ttt aaa tat caa agc ctt cta gta tct tct ggc      816
   Ser Ile Trp Ser Leu Phe Lys Tyr Gln Ser Leu Leu Val Ser Ser Gly
               260                 265                 270
   gct aat tta tat gca agc ggt agt gga cca cag cag act caa tca ttt      864
   Ala Asn Leu Tyr Ala Ser Gly Ser Gly Pro Gln Gln Thr Gln Ser Phe
           275                 280                 285
   act tca caa gac tgg cca ttt tta tat tct ctt ttc caa gtt aat tca      912
   Thr Ser Gln Asp Trp Pro Phe Leu Tyr Ser Leu Phe Gln Val Asn Ser
       290                 295                 300
   aat tat gtg tta aat ggc ttt agt ggc gct aga ctt acg cag act ttc      960
   Asn Tyr Val Leu Asn Gly Phe Ser Gly Ala Arg Leu Thr Gln Thr Phe
   305                 310                 315                 320
   cct aat att ggt ggt tta cct ggt act act aca act cac gca ttg ctt     1008
   Pro Asn Ile Gly Gly Leu Pro Gly Thr Thr Thr Thr His Ala Leu Leu
                   325                 330                 335
   gcg gca agg gtc aat tac agt gga gga gtt tcg tct ggt gat ata ggc     1056
   Ala Ala Arg Val Asn Tyr Ser Gly Gly Val Ser Ser Gly Asp Ile Gly
               340                 345                 350
   gct gtg ttt aat caa aat ttt agt tgt agc aca ttt ctc cca cct ttg      1104
   Ala Val Phe Asn Gln Asn Phe Ser Cys Ser Thr Phe Leu Pro Pro Leu
           355                 360                 365
   tta aca cca ttt gtt agg agt tgg cta gat tca ggt tca gat cga ggg      1152
   Leu Thr Pro Phe Val Arg Ser Trp Leu Asp Ser Gly Ser Asp Arg Gly
       370                 375                 380
   ggt gtt aat acc gtt aca aat tgg caa aca gaa tcg ttt gag tca act      1200
   Gly Val Asn Thr Val Thr Asn Trp Gln Thr Glu Ser Phe Glu Ser Thr
   385                 390                 395                 400
   tta ggt tta agg tgt ggt gct ttt aca gct cgt ggt aat tca aac tat      1248
   Leu Gly Leu Arg Cys Gly Ala Phe Thr Ala Arg Gly Asn Ser Asn Tyr
                   405                 410                 415
   ttc cca gat tat ttt atc cgt aat att tca gga gtt cct tta gtt gtt      1296
   Phe Pro Asp Tyr Phe Ile Arg Asn Ile Ser Gly Val Pro Leu Val Val
               420                 425                 430
   aga aat gaa gat tta aga aga ccg tta cac tat aat gaa ata aga aat      1344
   Arg Asn Glu Asp Leu Arg Arg Pro Leu His Tyr Asn Glu Ile Arg Asn
           435                 440                 445
   ata gaa agt cct tca gga aca cct ggt gga tta cga gct tat atg gta      1392
   Ile Glu Ser Pro Ser Gly Thr Pro Gly Gly Leu Arg Ala Tyr Met Val
       450                 455                 460
   tct gtg cat aat aga aaa aat aat atc tat gcc gtg cat gaa aat ggt      1440
   Ser Val His Asn Arg Lys Asn Asn Ile Tyr Ala Val His Glu Asn Gly
   465                 470                 475                 480
   act atg att cat tta gcg ccg gaa gat tat aca gga ttc acc ata tcg      1488
   Thr Met Ile His Leu Ala Pro Glu Asp Tyr Thr Gly Phe Thr Ile Ser
                   485                 490                 495
   ccg ata cat gca act caa gtg aat aat caa acg cga aca ttt att tct      1536
   Pro Ile His Ala Thr Gln Val Asn Asn Gln Thr Arg Thr Phe Ile Ser
               500                 505                 510
   gaa aaa ttt gga aat caa ggt gat tcc tta aga ttt gaa caa agc aac      1584
   Glu Lys Phe Gly Asn Gln Gly Asp Ser Leu Arg Phe Glu Gln Ser Asn
           515                 520                 525
   acg aca gca cgt tat aca ctt aga gga aat gga aat agt tac aat ctt      1632
   Thr Thr Ala Arg Tyr Thr Leu Arg Gly Asn Gly Asn Ser Tyr Asn Leu
       530                 535                 540
   tat tta aga gta tct tca cta gga aat tcc act att cga gtt act ata      1680
   Tyr Leu Arg Val Ser Ser Leu Gly Asn Ser Thr Ile Arg Val Thr Ile
   545                 550                 555                 560
   aac ggt agg gtt tat act gct tca aat gtt aat act act aca aat aac      1728
   Asn Gly Arg Val Tyr Thr Ala Ser Asn Val Asn Thr Thr Thr Asn Asn
                   565                 570                 575
   gat gga gtt aat gat aat ggc gct cgt ttt tta gat att aat atg ggt      1776
   Asp Gly Val Asn Asp Asn Gly Ala Arg Phe Leu Asp Ile Asn Met Gly
               580                 585                 590
   aat gta gta gca agt gat aat act aat gta ccg tta gat ata aat gtg      1824
   Asn Val Val Ala Ser Asp Asn Thr Asn Val Pro Leu Asp Ile Asn Val
           595                 600                 605
   aca ttt aac tcc ggt act caa ttt gag ctt atg aat att atg ttt gtt      1872
   Thr Phe Asn Ser Gly Thr Gln Phe Glu Leu Met Asn Ile Met Phe Val
       610                 615                 620
   cca act aat ctt cca cca ata tat taa                                  1899
   Pro Thr Asn Leu Pro Pro Ile Tyr
   625                 630
<210> 2
   <211> 632
   <212> PRT
   <213> Bacillus thuringiensis
<400> 2
   Met Asn Asn Val Leu Asn Asn Gly Arg Thr Thr Ile Cys Asp Ala Tyr
   1               5                   10                  15
   Asn Val Val Ala His Asp Pro Phe Ser Phe Glu His Lys Ser Leu Asp
               20                  25                  30
   Thr Ile Arg Lys Glu Trp Met Glu Trp Lys Arg Thr Asp His Ser Leu
           35                  40                  45
   Tyr Val Ala Pro Ile Val Gly Thr Val Ser Ser Phe Leu Leu Lys Lys
       50                  55                  60
   Val Gly Ser Leu Ile Gly Lys Arg Ile Leu Ser Glu Leu Trp Gly Leu
   65                  70                  75                  80
   Ile Phe Pro Ser Gly Ser Thr Asn Leu Met Gln Asp Ile Leu Arg Glu
                   85                  90                  95
   Thr Glu Gln Phe Leu Asn Gln Arg Leu Asn Thr Asp Thr Leu Ala Arg
               100                 105                 110
   Val Asn Ala Glu Leu Glu Gly Leu Gln Ala Asn Ile Arg Glu Phe Asn
           115                 120                 125
   Gln Gln Val Asp Asn Phe Leu Asn Pro Thr Gln Asn Pro Val Pro Leu
   130 135 140
   Ser Ile Thr Ser Ser Val Asn Thr Met Gln Gln Leu Phe Leu Asn Arg
   145 150 155 160
   Leu Pro Gln Phe Arg Val Gln Gly Tyr Gln Leu Leu Leu Leu Pro Leu
   165 170 175
   Phe Ala Gln Ala Ala Asn Met His Leu Ser Phe Ile Arg Asp Val Val
   180 185 190
   Leu Asn Ala Asp Glu Trp Gly Ile Ser Ala Ala Thr Leu Arg Thr Tyr
   195 200 205
   Gln Asn Tyr Leu Lys Asn Tyr Thr Thr Glu Tyr Ser Asn Tyr Cys Ile
   210 215 220
   Asn Thr Tyr Gln Thr Ala Phe Arg Gly Leu Asn Thr Arg Leu His Asp
   225 230 235 240
   Met Leu Glu Phe Arg Thr Tyr Met Phe Leu Asn Val Phe Glu Tyr Val
   245 250 255
   Ser Ile Trp Ser Leu Phe Lys Tyr Gln Ser Leu Leu Val Ser Ser Gly
   260 265 270
   Ala Asn Leu Tyr Ala Ser Gly Ser Gly Pro Gln Gln Thr Gln Ser Phe
   275 280 285
   Thr Ser Gln Asp Trp Pro Phe Leu Tyr Ser Leu Phe Gln Val Asn Ser
   290 295 300
   Asn Tyr Val Leu Asn Gly Phe Ser Gly Ala Arg Leu Thr Gln Thr Phe
   305 310 315 320
   Pro Asn Ile Gly Gly Leu Pro Gly Thr Thr Thr Thr His Ala Leu Leu
   325 330 335
   Ala Ala Arg Val Asn Tyr Ser Gly Gly Val Ser Ser Gly Asp Ile Gly
   340 345 350
   Ala Val Phe Asn Gln Asn Phe Ser Cys Ser Thr Phe Leu Pro Pro Leu
           355                 360                 365
   Leu Thr Pro Phe Val Arg Ser Trp Leu Asp Ser Gly Ser Asp Arg Gly
       370                 375                 380
   Gly Val Asn Thr Val Thr Asn Trp Gln Thr Glu Ser Phe Glu Ser Thr
   385                 390                 395                 400
   Leu Gly Leu Arg Cys Gly Ala Phe Thr Ala Arg Gly Asn Ser Asn Tyr
                   405                 410                 415
   Phe Pro Asp Tyr Phe Ile Arg Asn Ile Ser Gly Val Pro Leu Val Val
               420                 425                 430
   Arg Asn Glu Asp Leu Arg Arg Pro Leu His Tyr Asn Glu Ile Arg Asn
           435                 440                 445
   Ile Glu Ser Pro Ser Gly Thr Pro Gly Gly Leu Arg Ala Tyr Met Val
       450                 455                 460
   Ser Val His Asn Arg Lys Asn Asn Ile Tyr Ala Val His Glu Asn Gly
   465                 470                 475                 480
   Thr Met Ile His Leu Ala Pro Glu Asp Tyr Thr Gly Phe Thr Ile Ser
                   485                 490                 495
   Pro Ile His Ala Thr Gln Val Asn Asn Gln Thr Arg Thr Phe Ile Ser
               500                 505                 510
   Glu Lys Phe Gly Asn Gln Gly Asp Ser Leu Arg Phe Glu Gln Ser Asn
           515                 520                 525
   Thr Thr Ala Arg Tyr Thr Leu Arg Gly Asn Gly Asn Ser Tyr Asn Leu
       530                 535                 540
   Tyr Leu Arg Val Ser Ser Leu Gly Asn Ser Thr Ile Arg Val Thr Ile
   545                 550                 555                 560
   Asn Gly Arg Val Tyr Thr Ala Ser Asn Val Asn Thr Thr Thr Asn Asn
                   565                 570                 575
   Asp Gly Val Asn Asp Asn Gly Ala Arg Phe Leu Asp Ile Asn Met Gly
   580 585 590
   Asn Val Val Ala Ser Asp Asn Thr Asn Val Pro Leu Asp Ile Asn Val
   595 600 605
   Thr Phe Asn Ser Gly Thr Gln Phe Glu Leu Met Asn Ile Met Phe Val
   610 615 620
   Pro Thr Asn Leu Pro Pro Ile Tyr
   625 630
<210> 3
   <211> 1910
   <212> DNA
   <213> Artificial
<220>
   <223> artificial cry2Ae DNA sequence for expression in cotton
<220>
   <221> CDS
   <222> (3)..(1901)
<400> 3
   cc atg gct aac aac gtt ctt aac aac ggt agg act act att tgc gat 47
   Met Ala Asn Asn Val Leu Asn Asn Gly Arg Thr Thr Ile Cys Asp
   1 5 10 15
   gca tac aac gtt gtt gct cat gat cct ttc tct ttc gag cat aag tct 95
   Ala Tyr Asn Val Val Ala His Asp Pro Phe Ser Phe Glu His Lys Ser
   20 25 30
   ctt gat aca att agg aag gag tgg atg gag tgg aag agg act gat cat 143
   Leu Asp Thr Ile Arg Lys Glu Trp Met Glu Trp Lys Arg Thr Asp His
   35 40 45
   tct ctt tac gtt gct cct att gtt ggt act gtt tct tct ttc ctt ctt 191
   Ser Leu Tyr Val Ala Pro Ile Val Gly Thr Val Ser Ser Phe Leu Leu
   50 55 60
   aag aag gtt ggt tct ctt atc ggt aag agg atc ctt tct gag ctt tgg 239
   Lys Lys Val Gly Ser Leu Ile Gly Lys Arg Ile Leu Ser Glu Leu Trp
   65 70 75
   ggt ctt atc ttc cct tct ggt tct act aac ctt atg caa gat att ctt 287
   Gly Leu Ile Phe Pro Ser Gly Ser Thr Asn Leu Met Gln Asp Ile Leu
   80 85 90 95
   agg gag act gaa caa ttc ctt aac cag agg ctt aac act gat act ctt 335
   Arg Glu Thr Glu Gln Phe Leu Asn Gln Arg Leu Asn Thr Asp Thr Leu
   100 105 110
   gct agg gtt aac gct gag ctt gag ggt ctt caa gct aac att agg gaa      383
   Ala Arg Val Asn Ala Glu Leu Glu Gly Leu Gln Ala Asn Ile Arg Glu
           115                 120                 125
   ttc aac cag caa gtt gat aac ttc ctt aac cct act caa aac cct gtt      431
   Phe Asn Gln Gln Val Asp Asn Phe Leu Asn Pro Thr Gln Asn Pro Val
       130                 135                 140
   cct ctt tct att act tct tct gtt aac act atg caa caa ctt ttc ctt      479
   Pro Leu Ser Ile Thr Ser Ser Val Asn Thr Met Gln Gln Leu Phe Leu
   145                 150                 155
   aac agg ctt cct caa ttc agg gtt caa ggt tac caa ctt ctt ctt ctt      527
   Asn Arg Leu Pro Gln Phe Arg Val Gln Gly Tyr Gln Leu Leu Leu Leu
   160                 165                 170                 175
   cct ctt ttc gct caa gct gct aac atg cac cta agc ttc att agg gat      575
   Pro Leu Phe Ala Gln Ala Ala Asn Met His Leu Ser Phe Ile Arg Asp
                   180                 185                 190
   gtt gtt ctt aac gct gat gag tgg ggt att tct gct gct act ctt agg      623
   Val Val Leu Asn Ala Asp Glu Trp Gly Ile Ser Ala Ala Thr Leu Arg
           195                 200                 205
   act tac caa aac tac ctt aag aac tac act act gag tac tct aac tac      671
   Thr Tyr Gln Asn Tyr Leu Lys Asn Tyr Thr Thr Glu Tyr Ser Asn Tyr
       210                 215                 220
   tgc att aac act tac caa act gct ttc agg ggt ctt aac act agg ctt      719
   Cys Ile Asn Thr Tyr Gln Thr Ala Phe Arg Gly Leu Asn Thr Arg Leu
   225                 230                 235
   cat gat atg ctt gag ttc agg act tac atg ttc ctt aac gtt ttc gag      767
   His Asp Met Leu Glu Phe Arg Thr Tyr Met Phe Leu Asn Val Phe Glu
   240                 245                 250                 255
   tac gtt tct att tgg tct ctt ttc aag tac cag tct ctt ctt gtt tct      815
   Tyr Val Ser Ile Trp Ser Leu Phe Lys Tyr Gln Ser Leu Leu Val Ser
                   260                 265                 270
   tct ggt gct aac ctt tac gct tct ggt tct ggt cct caa caa act caa      863
   Ser Gly Ala Asn Leu Tyr Ala Ser Gly Ser Gly Pro Gln Gln Thr Gln
           275                 280                 285
   tct ttc act tct caa gac tgg cct ttc ctt tac tct ctt ttc caa gtt      911
   Ser Phe Thr Ser Gln Asp Trp Pro Phe Leu Tyr Ser Leu Phe Gln Val
       290                 295                 300
   aac tct aac tac gtt ctt aac ggt ttc tct ggt gct agg ctt act caa      959
   Asn Ser Asn Tyr Val Leu Asn Gly Phe Ser Gly Ala Arg Leu Thr Gln
   305                 310                 315
   act ttc cct aac atc ggt ggt ctt cct ggt act act act act cat gct     1007
   Thr Phe Pro Asn Ile Gly Gly Leu Pro Gly Thr Thr Thr Thr His Ala
   320                 325                 330                 335
   ctt ctt gct gct agg gtt aac tac tct ggt ggt gtt tct tct ggt gat      1055
   Leu Leu Ala Ala Arg Val Asn Tyr Ser Gly Gly Val Ser Ser Gly Asp
                   340                 345                 350
   atc ggt gct gtt ttc aac cag aac ttc tct tgc tct act ttc ctt cct      1103
   Ile Gly Ala Val Phe Asn Gln Asn Phe Ser Cys Ser Thr Phe Leu Pro
               355                 360                 365
   cct ctt ctt act cct ttc gtt agg tct tgg ctt gat tct ggt tct gat      1151
   Pro Leu Leu Thr Pro Phe Val Arg Ser Trp Leu Asp Ser Gly Ser Asp
           370                 375                 380
   agg ggt ggt gtt aac act gtt act aac tgg caa act gag tct ttc gag      1199
   Arg Gly Gly Val Asn Thr Val Thr Asn Trp Gln Thr Glu Ser Phe Glu
       385                 390                 395
   tct act ctt ggt ctt agg tgc ggt gct ttc act gct agg ggt aac tct      1247
   Ser Thr Leu Gly Leu Arg Cys Gly Ala Phe Thr Ala Arg Gly Asn Ser
   400                 405                 410                 415
   aac tac ttc cct gat tac ttc att agg aac att tct ggt gtt cct ctt      1295
   Asn Tyr Phe Pro Asp Tyr Phe Ile Arg Asn Ile Ser Gly Val Pro Leu
                   420                 425                 430
   gtt gtt agg aac gag gat ctt agg agg cct ctt cat tac aac gag att      1343
   Val Val Arg Asn Glu Asp Leu Arg Arg Pro Leu His Tyr Asn Glu Ile
               435                 440                 445
   agg aac att gag tct cct tct ggt act cct ggt ggt ctt agg gct tac      1391
   Arg Asn Ile Glu Ser Pro Ser Gly Thr Pro Gly Gly Leu Arg Ala Tyr
           450                 455                 460
   atg gtt tct gtt cat aac agg aag aac aac atc tac gct gtt cat gag      1439
   Met Val Ser Val His Asn Arg Lys Asn Asn Ile Tyr Ala Val His Glu
       465                 470                 475
   aac ggt act atg att cat ctt gct cct gag gat tac acc ggt ttc acc      1487
   Asn Gly Thr Met Ile His Leu Ala Pro Glu Asp Tyr Thr Gly Phe Thr
   480                 485                 490                 495
   atc tcc ccc atc cac gcc acc cag gtc aat aat cag acc agg acc ttc      1535
   Ile Ser Pro Ile His Ala Thr Gln Val Asn Asn Gln Thr Arg Thr Phe
                   500                 505                 510
   atc tcc gag aag ttc ggc aac cag ggc gac tcc ctg agg ttc gag cag      1583
   Ile Ser Glu Lys Phe Gly Asn Gln Gly Asp Ser Leu Arg Phe Glu Gln
               515                 520                 525
   tcc aac acc acc gcc agg tac acc ctg agg ggc aac ggc aac tcc tac      1631
   Ser Asn Thr Thr Ala Arg Tyr Thr Leu Arg Gly Asn Gly Asn Ser Tyr
           530                 535                 540
   aac ctg tac ctc agg gtg tcc tcc ctc ggc aac tcc acc atc agg gtc      1679
   Asn Leu Tyr Leu Arg Val Ser Ser Leu Gly Asn Ser Thr Ile Arg Val
       545                 550                 555
   acc atc aac ggc agg gtg tac acc gcc tcc aac gtg aac acc acc acc      1727
   Thr Ile Asn Gly Arg Val Tyr Thr Ala Ser Asn Val Asn Thr Thr Thr
   560                 565                 570                 575
   aac aac gac ggc gtc aac gac aac ggc gct agg ttc ctg gac atc aac      1775
   Asn Asn Asp Gly Val Asn Asp Asn Gly Ala Arg Phe Leu Asp Ile Asn
                   580                 585                 590
   atg ggc aac gtc gtg gcc tcc gac aac acc aac gtg ccc ctg gac atc      1823
   Met Gly Asn Val Val Ala Ser Asp Asn Thr Asn Val Pro Leu Asp Ile
               595                 600                 605
   aac gtg aca ttt aac tcc ggc acc cag ttc gag ctg atg aac atc atg      1871
   Asn Val Thr Phe Asn Ser Gly Thr Gln Phe Glu Leu Met Asn Ile Met
           610                 615                 620
   ttc gtg cca act aac ctc cca ccc atc tac tgagctagc                    1910
   Phe Val Pro Thr Asn Leu Pro Pro Ile Tyr
       625                 630
<210> 4
   <211> 633
   <212> PRT
   <213> Artificial
<400> 4
   Met Ala Asn Asn Val Leu Asn Asn Gly Arg Thr Thr Ile Cys Asp Ala
   1               5                   10                  15
   Tyr Asn Val Val Ala His Asp Pro Phe Ser Phe Glu His Lys Ser Leu
               20                  25                  30
   Asp Thr Ile Arg Lys Glu Trp Met Glu Trp Lys Arg Thr Asp His Ser
           35                  40                  45
   Leu Tyr Val Ala Pro Ile Val Gly Thr Val Ser Ser Phe Leu Leu Lys
       50                  55                  60
   Lys Val Gly Ser Leu Ile Gly Lys Arg Ile Leu Ser Glu Leu Trp Gly
   65                  70                  75                  80
   Leu Ile Phe Pro Ser Gly Ser Thr Asn Leu Met Gln Asp Ile Leu Arg
                   85                  90                  95
   Glu Thr Glu Gln Phe Leu Asn Gln Arg Leu Asn Thr Asp Thr Leu Ala
               100                 105                 110
   Arg Val Asn Ala Glu Leu Glu Gly Leu Gln Ala Asn Ile Arg Glu Phe
   115 120 125
   Asn Gln Gln Val Asp Asn Phe Leu Asn Pro Thr Gln Asn Pro Val Pro
   130 135 140
   Leu Ser Ile Thr Ser Ser Val Asn Thr Met Gln Gln Leu Phe Leu Asn
   145 150 155 160
   Arg Leu Pro Gln Phe Arg Val Gln Gly Tyr Gln Leu Leu Leu Leu Pro
   165 170 175
   Leu Phe Ala Gln Ala Ala Asn Met His Leu Ser Phe Ile Arg Asp Val
   180 185 190
   Val Leu Asn Ala Asp Glu Trp Gly Ile Ser Ala Ala Thr Leu Arg Thr
   195 200 205
   Tyr Gln Asn Tyr Leu Lys Asn Tyr Thr Thr Glu Tyr Ser Asn Tyr Cys
   210 215 220
   Ile Asn Thr Tyr Gln Thr Ala Phe Arg Gly Leu Asn Thr Arg Leu His
   225 230 235 240
   Asp Met Leu Glu Phe Arg Thr Tyr Met Phe Leu Asn Val Phe Glu Tyr
   245 250 255
   Val Ser Ile Trp Ser Leu Phe Lys Tyr Gln Ser Leu Leu Val Ser Ser
   260 265 270
   Gly Ala Asn Leu Tyr Ala Ser Gly Ser Gly Pro Gln Gln Thr Gln Ser
   275 280 285
   Phe Thr Ser Gln Asp Trp Pro Phe Leu Tyr Ser Leu Phe Gln Val Asn
   290 295 300
   Ser Asn Tyr Val Leu Asn Gly Phe Ser Gly Ala Arg Leu Thr Gln Thr
   305 310 315 320
   Phe Pro Asn Ile Gly Gly Leu Pro Gly Thr Thr Thr Thr His Ala Leu
   325 330 335
   Leu Ala Ala Arg Val Asn Tyr Ser Gly Gly Val Ser Ser Gly Asp Ile
   340 345 350
   Gly Ala Val Phe Asn Gln Asn Phe Ser Cys Ser Thr Phe Leu Pro Pro
   355 360 365
   Leu Leu Thr Pro Phe Val Arg Ser Trp Leu Asp Ser Gly Ser Asp Arg
   370 375 380
   Gly Gly Val Asn Thr Val Thr Asn Trp Gln Thr Glu Ser Phe Glu Ser
   385 390 395 400
   Thr Leu Gly Leu Arg Cys Gly Ala Phe Thr Ala Arg Gly Asn Ser Asn
   405 410 415
   Tyr Phe Pro Asp Tyr Phe Ile Arg Asn Ile Ser Gly Val Pro Leu Val
   420 425 430
   Val Arg Asn Glu Asp Leu Arg Arg Pro Leu His Tyr Asn Glu Ile Arg
   435 440 445
   Asn Ile Glu Ser Pro Ser Gly Thr Pro Gly Gly Leu Arg Ala Tyr Met
   450 455 460
   Val Ser Val His Asn Arg Lys Asn Asn Ile Tyr Ala Val His Glu Asn
   465 470 475 480
   Gly Thr Met Ile His Leu Ala Pro Glu Asp Tyr Thr Gly Phe Thr Ile
   485 490 495
   Ser Pro Ile His Ala Thr Gln Val Asn Asn Gln Thr Arg Thr Phe Ile
   500 505 510
   Ser Glu Lys Phe Gly Asn Gln Gly Asp Ser Leu Arg Phe Glu Gln Ser
   515 520 525
   Asn Thr Thr Ala Arg Tyr Thr Leu Arg Gly Asn Gly Asn Ser Tyr Asn
   530 535 540
   Leu Tyr Leu Arg Val Ser Ser Leu Gly Asn Ser Thr Ile Arg Val Thr
   545 550 555 560
   Ile Asn Gly Arg Val Tyr Thr Ala Ser Asn Val Asn Thr Thr Thr Asn
   565 570 575
   Asn Asp Gly Val Asn Asp Asn Gly Ala Arg Phe Leu Asp Ile Asn Met
   580 585 590
   Gly Asn Val Val Ala Ser Asp Asn Thr Asn Val Pro Leu Asp Ile Asn
   595 600 605
   Val Thr Phe Asn Ser Gly Thr Gln Phe Glu Leu Met Asn Ile Met Phe
   610 615 620
   Val Pro Thr Asn Leu Pro Pro Ile Tyr
   625 630
<210> 5
   <211> 1910
   <212> DNA
   <213> Artificial
<220>
   <223> artificial cry2Ae DNA sequence for expression in corn
<400> 5
   ccatggctaa caacgtgctg aacaacggca ggaccaccat ctgcgatgca tacaacgtgg 60
   tggcccacga cccattcagc ttcgagcaca agagcctgga caccatccgc aaggagtgga 120
   tggagtggaa gcgcaccgac cacagcctgt acgtggcccc tatcgtgggc accgtgagca 180
   gcttcctgct gaagaaggtg ggcagcctga tcggcaagag gatcctgagc gagctgtggg 240
   gcctgatctt cccaagcggc agcaccaacc tgatgcagga catcctgagg gagaccgagc 300
   agttcctgaa ccagcgcctg aacaccgaca ccctggctcg cgtgaacgcc gagctggagg 360
   gcctccaggc caacatcagg gaattcaacc agcaggtgga caacttcctg aacccaaccc 420
   agaacccagt gccactgagc atcaccagca gcgtgaacac catgcagcag ctgttcctga 480
   accgcctgcc acagttccgc gtgcagggct accagctgct gctgctgcca ctgttcgccc 540
   aggctgccaa catgcaccta agcttcatcc gcgacgtggt gctgaacgcc gacgagtggg 600
   gcatcagcgc tgccaccctg cgcacctacc agaactacct gaagaactac accaccgagt 660
   acagcaacta ctgcatcaac acctaccaga ccgccttcag gggcctgaac accaggctgc 720
   acgacatgct ggagttccgc acctacatgt tcctgaacgt gttcgagtac gtgagcatct 780
   ggagcctgtt caagtaccag agcctgctgg tgagcagcgg tgccaacctg tacgccagcg 840
   gcagcggtcc acagcagacc cagagcttca ccagccagga ctggcccttc ctgtacagcc 900
   tgttccaggt gaacagcaac tacgtgctga acggcttcag cggtgccagg ctgacccaga 960
   ccttcccaaa catcggaggc ctgccaggca ccaccaccac ccacgccctg ctggctgcca 1020
   gggtgaacta cagcggtggc gtgagcagcg gcgatatcgg cgctgtgttc aaccagaact 1080
   tcagctgcag caccttcctg ccaccactgc tgaccccatt cgtgcgcagc tggctggaca 1140
   gcggcagcga caggggtggc gtgaacaccg tgaccaactg gcagaccgag agcttcgaga 1200
   gcaccctggg cctgcgctgc ggtgccttca ccgccagggg caacagcaac tacttcccag 1260
   actacttcat ccgcaacatc agcggcgtgc cactggtggt gcgcaacgag gacctgcgca 1320
   ggccactgca ctacaacgag atccgcaaca tcgagagccc aagcggcacc ccaggaggcc 1380
   tgagggccta catggtgagc gtgcacaacc gcaagaacaa catctacgcc gtgcacgaga 1440
   acggcaccat gatccacctg gccccagagg actacaccgg tttcaccatc tcccccatcc 1500
   acgccaccca ggtcaataat cagaccagga ccttcatctc cgagaagttc ggcaaccagg 1560
   gcgactccct gaggttcgag cagtccaaca ccaccgccag gtacaccctg aggggcaacg 1620
   gcaactccta caacctgtac ctcagggtgt cctccctcgg caactccacc atcagggtca 1680
   ccatcaacgg cagggtgtac accgcctcca acgtgaacac caccaccaac aacgacggcg 1740
   tcaacgacaa cggcgctagg ttcctggaca tcaacatggg caacgtcgtg gcctccgaca 1800
   acaccaacgt gcccctggac atcaacgtga catttaactc cggcacccag ttcgagctga 1860
   tgaacatcat gttcgtgcca actaacctcc cacccatcta ctgagctagc 1910

## Description

### INTRODUCTION

The present invention relates to new nucleic acid sequences, particularly DNA sequences, encoding insecticidal proteins produced by *Bacillus thuringiensis* strains. Particularly, new nucleic acid sequences, particularly DNA sequences encoding proteins designated as Cry2Ae are provided which are useful to protect plants from insect damage. Also included herein are micro-organisms and plants transformed with a nucleic acid sequence, particularly a DNA sequence, encoding at least one of the newly isolated Cry2A proteins.

### BACKGROUND OF THE INVENTION

### (i) Field of the Invention:

*Bacillus thuringiensis* (abbreviated herein as "Bt") is well known for its specific toxicity to insect pests, and has been used since almost a century to control insect pests of plants. In more recent years, transgenic plants expressing *Bt* proteins were made which were found to successfully control insect damage on plants (e.g., Vaeck et al., 1987, Jansens et al., 1997).
Despite the isolation of quite a number of insecticidal *Bt* genes, the search for new genes encoding insecticidal proteins continues. Indeed, Insecticidal Bt proteins are known to have a relatively narrow target insect range compared to chemical insecticides. Also, having multiple toxins to the same target insect species allows the use of proteins having different modes of action so that insect resistance development can be prevented or delayed. And, insecticidal Bt proteins with different amino acid sequences have different levels of insecticidal efficacy against specific insects, making it desirable to have several different insecticidal proteins available in order to be able to control the relevant insect pests of different crop plants.

### (ii) Description of Related Art:

Previously, several types of Cry2A-proteins were identified (see Crickmore et al., 1998).
The new Cry2Ae protein of this invention has the highest amino acid sequence identity to the Cry2Aa1 protein (Donovan et al., GenBank accession number M31738), but still differs in about 9 percent of its amino acid sequence.
The closest sequence identity to the Cry2Af protein was found in the Cry2Ab1 protein (Widner and Whiteley, GenBank accession number M23724), but both proteins still differ in about 5 percent of their amino acid sequence.
The closest sequence identity to the Cry2Ag protein was found in the Cry2Ac1 protein (Wu et al., GenBank accession number X57252), but both proteins still differ in about 20 percent of their amino acid sequence.
Further known Cry2A proteins include the Cry2Ad1 protein (Choi et al., 1999), and other Cry2Aa, Cry2Ab, and Cry2Ac proteins (Crickmore et al., 1998). Cry2A-like proteins and DNA sequences encoding them are also shown in US patent 5,338,544, in published PCT patent application WO 00/26371 and in published PCT patent application WO 98/40490.
Expression of Cry2A-type proteins in plants has been described, e.g., in Kota et al. (1999) and in published PCT patent application WO 00/26371.

Widner and Whiteley (1989, J. Bacteriol. 171, 965-974) describe the cryB1 and cryB2 genes, encoding proteins which are now known as Cry2Aa and Cry2Ab, respectively (see, Crickmore et al., 1998).

Published PCT patent application WO01/19859 (which is part of the state of the art under Art 54(3)(4) EPC) describes a cry2Ae gene and protein in SEQ ID No. 1 and 2, respectively.

### OBJECTS AND SUMMARY OF THE INVENTION

In accordance with this invention is provided a nucleic acid sequence, particularly a DNA sequence, encoding a protein comprising the amino acid sequence of the smallest toxic fragment of the protein encoded by the *cry*2*Ae* gene deposited at the BCCM-LMBP under accession number LMBP 4248.

Particularly preferred in accordance with this invention is a nucleic acid sequence, particularly a DNA sequence, encoding a protein comprising the amino acid sequence of an insecticidal fragment of the protein of SEQ ID No. 2.

Further, in accordance with this invention are provided nucleic acid sequences, particularly DNA sequences, encoding a protein comprising the amino acid sequence of SEQ ID No. 2 from amino acid position 1 to amino acid position 632.

Further, in accordance with this invention are provided the above nucleic acid sequences, particularly DNA sequences, comprising an artificial sequence, having a different codon usage compared to the naturally occurring sequence, but encoding the same protein or its insecticidal fragment, preferably such codon usage resembles that of plants, particularly the host plant in which the nucleic acid sequence, particularly the DNA, is to be transformed.

Even further provided in accordance with this invention is a protein comprising the amino acid sequence of the smallest toxic fragment of the protein encoded by the *cry*2*Ae* gene deposited at the BCCM-LMBP under accession number LMBP 4248.

Particularly preferred herein is a protein comprising the amino acid sequence of an insecticidal fragment of the protein of SEQ ID No. 2.

Also provided herein are chimeric genes comprising the DNA as defined above under the control of a plant-expressible promoter, and plant cells, plants or seeds transformed to contain those chimeric genes, particularly plant cells, plants, or seeds selected from the group consisting of: corn, cotton, rice, tobacco, oilseed rape, Brassica species, eggplant soybean, potato, sunflower, tomato, sugarcane, tea, beans, tobacco, strawberry, clover, cucumber, watermelon, pepper, oat, barley, wheat, dahlia, gladiolus, chrysanthemum, sugarbeet, sorghum, alfalfa, apple, pear, strawberry, and peanut. In accordance with this invention, the chimeric gene can be integrated in the nuclear, plastid or mitochondrial DNA of the plant cells, or can also contain a DNA encoding an effective targeting or transit peptide for targeting to the vacuole, chloroplast, mitochondrium, plastid, or for secretion.

Further in accordance with this invention are provided micro-organisms, transformed to contain any of the above DNA sequences, particularly those selected from the genus *Pseudomonas, Agrobacterium, Escherichia,* or *Bacillus.*

Also provided herein is a process for controlling insects, comprising expressing any of the above nucleic acid sequences, particularly DNA sequences, in a host cell, particularly plant cells, and contacting insects with said host cells, and a process for rendering a plant resistant to insects, comprising transforming plants cells with any of the above DNA sequences or chimeric genes, and regenerating transformed plants from such cells which are resistant to insects.

This invention also relates to a method for controlling lepidopteran insects, particularly lepidopteran insect pests of cotton, corn or soybean, which method comprises applying to an area or plant to be protected, a Cry2Ae protein as defined herein, (i.e., by planting a plant transformed with a *cryr2Ae* gene of this invention, or by spraying a composition containing a Cry2Ae protein of this invention). The invention also relates to the use of the Cry2Ae protein, against Lepidopteran insect pests to minimize damage to soybean plants.

This invention further relates to a method for controlling lepidopteran rice insect pests, particularly Lepidopteran rice stemborers, rice skippers, rice cutworms, rice armyworms, rice caseworms or rice leaffolders, preferably an insect selected from the group consisting of: Chilo suppressalis, Chilo partellus, Scirpophaga incertulas, Sesamia inferens, Cnaphalocrocis medinalis, Marasmia patnalis, Marasmia exigua, Marasmia ruralis, Scirpophaga innotata, which method comprises applying to an area or plant to be protected, a Cry2Ae protein as defined herein, (i.e., by planting a rice plant transformed with a *cry*2*Ae* gene of this invention, or spraying a composition containing a Cry2Ae protein of this invention). The invention also relates to the use of the Cry2Ae protein, against Lepidopteran rice insect pests to minimize damage to rice plants.

### DETAILED DESCRIPTION OF PREFERRED EMBODIMENTS OF THE INVENTION

In accordance with this invention, a "nucleic acid sequence" refers to a DNA or RNA molecule in single or double stranded form, preferably a DNA or RNA, particularly a DNA, encoding any of the Cry2A proteins of this invention. An "isolated nucleic acid sequence", as used herein, refers to a nucleic acid sequence which is no longer in the natural environment where it was isolated from, e.g., the nucliec acid sequence in another bacterial host or in a plant nuclear genome.

In accordance with this invention, the terms "protein" or "polypeptide" are used interchangeably to refer to a sequence of amino acids, without reference to any functionality, size, three-dimensional structures or origin. Hence, a fragment or portion of a Cry2A protein of the invention is still referred to herein as a "protein".

In accordance with this invention, nucleic acid sequences, particularly DNA sequences, encoding new *Bt* Cry toxins have been isolated and characterized. The new gene was designated *cry*2*Ae* and its encoded proteins Cry2Ae.

In accordance with this invention "Cry2Ae protein" refers to any protein comprising the smallest fragment of the amino acid sequence of SEQ ID No. 2 which retains insecticidal activity (hereinafter referred to as "smallest toxic fragment"), particularly any protein comprising the amino acid sequence from the amino acid at position 1 to the amino acid at position 625, particularly to the amino acid at position 632 in SEQ ID No. 2. This includes hybrids or chimeric proteins comprising the smallest toxic protein fragment, as well as proteins containing at least one of the three domains of the protein of SEQ ID No. 2. Also included in this definition are variants of the amino acid sequence in SEQ ID No. 2. such as proteins having a sequence identity of at least 92 %, particularly at least 93 %, 95 %, 96 %, 97 %, 98 % or 99 % at the amino acid sequence level, as determined using pairwise alignments using the GAP program of the Wisconsin package of GCG (Madison, Wisconsin, USA, version 10.0; use GCG defaults within the GAP program; for the amino acid sequence comparisons, use the blosum62 scoring matrix), preferably proteins having some, preferably 5-10, particularly less than 5, amino acids added, replaced or deleted without significantly changing, preferably without changing, the insecticidal activity of the protein, e.g., the Cry2Ae protein of SEQ ID No. 4.

The term "DNA/protein comprising the sequence X", as used herein, refers to a DNA or protein including or containing at least the sequence X, so that other nucleotide or amino acid sequences can be included at the 5' (or N-terminal) and/or 3' (or C-terminal) end, e.g. (the nucleotide sequence of) a selectable marker protein as disclosed in EP 0193 259, (the nucleotide sequence of) a transit peptide, and/or a 5' or 3' leader sequence.

The "smallest toxic fragment" of a Cry protein of the invention, as used herein, is that smallest fragment or portion of a Cry protein retaining insecticidal activity that can be obtained by enzymatic, preferably trypsin or chymotrypsin, digestion of the full length Cry protein, or that smallest fragment or portion of a Cry protein retaining insecticidal activity that can be obtained by making nucleotide deletions in the DNA encoding a Cry protein. The N- and C-terminal amino acid sequence ends of the smallest toxic fragment are conveniently determined by amino acid sequence determination of the above fragments by techniques routinely available in the art. For the Cry2A protein fragments retaining insecticidal activity of this invention, typically N-terminal deletions can be made while little can be deleted at their C-terminal end. For the Cry2Ae proteins of the invention, it is expected that deletions up to amino acid position 825 at the C-terminus (i.e., the C-terminal amino acid would be the amino acid at position 625) can be done while conserving the insecticidal activity. It is expected that N-terminal deletions up to around amino acid position 50, preferably N-terminal deletions up to amino acid position 50 (i.e., the N-terminal amino acid would be position 50 of the sequences shown in the sequence listing) in the amino acid sequence of the three Cry2A proteins of this invention, retain most of their insecticidal activity against Lepidopteran insects.

As used herein, the terms "*cry*2*Ae* DNA" refers to any DNA sequence encoding the Cry2Ae protein, respectively, as defined above. This includes naturally occurring, artificial or synthetic DNA sequences encoding the proteins of SEQ ID Nos. 2 or its insecticidal fragments or variants as defined above. Also included herein are DNA sequences encoding insecticidal proteins which are similar enough to the coding regions of the genomic DNA sequences deposited or the sequences provided in the sequence listing so that they can (i.e., have the ability to) hybridize to these DNA sequences under stringent hybridization conditions. Stringent hybridization conditions, as used herein, refers particularly to the following conditions: immobilizing the relevant genomic DNA sequences on a filter, and prehybridizing the filters for either 1 to 2 hours in 60 % formamide, 5 % SSPE, 2x Denhardt's reagent and 0.1 % SDS at 42 °C or 1 to 2 hours in 6x SSC, 2xDenhardt's reagent and 0-1 % SDS at 68°C. The denatured (dig- or radio-)labeled probe is then added directly to the prehybridization fluid and incubation is carried out for 16 to 24 hours at the appropriate temperature mentioned above. After incubation, the filters are then washed for 30 minutes at room temperature in 2x SSC. 0.1 % SDS, followed by 2 washes of 30 minutes each at 68 °C in 0.5 x SSC and 0.1 % SDS. An autoradiograph is established by exposing the filters for 24 to 48 hours to X-ray film (Kodak XAR-2 or equivalent) at -70°C with an intensifying screen. Of course, equivalent conditions and parameters can be used in this process while still retaining the desired stringent hybridization conditions. Preferred variants of the *cry*2*Ae* DNA of this invention are a DNA encoding the insecticidal Cry2Ae protein variants described above, or a DNA sequence encoding an insecticidal protein with at least 92 %, preferably at least 93 to 97 %, particularly at least 98 % or at least 99 %, sequence identity to the coding sequence of SEQ ID No. 1. Particularly, such DNA sequences also hybridize under stringent hybridization conditions to the *cry*2*Ae* coding sequence deposited at the BCCM-LMBP under accession number LMBP 4248, or to the coding sequence of SEQ ID No. 1.

The sequence identities referred to above are calculated using the GAP program of the Wisconsin package of GCG (Madison, Wisconsin, USA) version 10.0 (GCG defaults are used, for these DNA sequence comparisons, the "nwsgapdna" scoring matrix is used), the stringent hybridization conditions are as defined above.

"Insecticidal activity" of a protein, as used herein, means the capacity of a protein to kill insects when such protein is fed to insects, preferably by expression in a recombinant host such as a plant. "Insect-controlling amounts" of a protein, as used herein, refers to an amount of protein which is sufficient to limit damage on a plant by insects feeding on such plant to commercially acceptable levels, e.g. by killing the insects or by inhibiting the insect development, fertility or growth in such a manner that they provide less damage to a plant and plant yield is not significantly adversely affected.
In accordance with this invention, insects susceptible to the new Cry proteins of the invention are contacted with this protein in insect-controlling amounts, preferably insecticidal amounts. Preferred target insects for the proteins of this invention are economically damaging insect pests of corn, cotton, rice and soybean plants, particularly In Northern and Southern American counties. Particularly preferred target insects for the Cry2A proteins of this invention, particularly the Cry2Ae protein, are Heliothis spp, Helicoverpa spp., Spodoptera spp., Sesamia spp., Anticarsia spp., Ostrinia spp., Chilo spp., Sesamia spp., Marasmia spp., Scirpophaga spp. and Cnaphalocrocis spp. insects, preferably, most preferably Heliothis virescens, Helicoverpa zea, Helicoverpa armigera, Anticarsia gemmatalls and Ostrinia nubilalis.

The terms "Cry2A protein", "Cry2A protein of this Invention", "Cry protein, or "Cry protein of this invention", as used herein, refers to any one of the new proteins isolated in accordance with this invention and identified and defined herein as Cry2Ae protein. A Cry protein, as used herein, can be a protein in the full length size, also named a protoxin, or can be in a truncated form as long as the insecticidal activity is retained, or can be a combination of different proteins in a hybrid or fusion protein. A "Cry protoxin" refers to the full length crystal protein as it is encoded by the naturally-occurring *Bt* DNA sequence, a "Cry toxin" refers to an insecticidal fragment thereof, particularly the smallest toxic fragment thereof, typically in the molecular weight range of about 50-65 kD, particularly about 60 kD, as determined by SDS-PAGE electrophoresis. A "*cry* gene", "*cry*2*A* gene", "*cry* DNA" or "*cry2A* DNA", as used herein, is a DNA sequence encoding a Cry protein in accordance with this invention, referring to the *cry2Ae* DNA sequences defined above.

The nucleic acid sequence, particularly DNA sequence, encoding the Cry proteins of this invention can be isolated in a conventional manner from the recombinant *E*. *coli* strains, deposited in accordance with the Budapest Treaty on October 6, 2000 at the Vakgroep voor Moleculaire Biologie-Plasmidencollectie, Universiteit Gent, K.L. Ledeganckstraat 35, B-9000 Gent, Belgium (hereinafter abbreviated as "BCCM-LMBP") under the following accession numbers: BCCM-LMBP 4248 for strain XL1Blue:pUC1099E/cy2clone7, which encodes the Cry2Ae protein. The DNA sequences encoding the Cry proteins of the invention can be isolated from these deposited strains using routine techniques, and can be inserted in expression vectors to produce high amounts of Cry proteins. The Cry proteins can be used to prepare specific monoclonal or polyclonal antibodies in a conventional manner (Höfte et al., 1988).

Also, DNA sequences for use in this invention can be synthetically made. Indeed, because of the degeneracy of the genetic code, some amino acid codons can be replaced by others without changing the amino acid sequence of the protein. Furthermore, some amino acids can be substituted by other equivalent amino acids without significantly changing, preferably without changing, the insecticidal activity of the protein. Also, changes in amino acid sequence or composition in regions of the molecule, different from those responsible for binding or pore formation are less likely to cause a difference in insecticidal activity of the protein. Equivalents of the DNA sequences of the invention include DNA sequences hybridizing to the DNA sequence of the Cry proteins of SEQ ID. No. 1 under stringent hybridization conditions and encoding a protein with the same insecticidal characteristics as the protein of this invention, or DNA sequences having a different codon usage compared to the native *cry2A* genes of this invention but which encode a protein with the same insecticidal activity and with substantially the same, preferably the same, amino acid sequence. Examples of codon-optimized DNA sequences for the Cry2Ae protein of this invention are found In SEQ ID Nos. 3 and 5. These DNA sequences were optimized by adapting the codon usage to that most preferred in plant genes, particularly to genes native to the plant genus or species of interest (Bennetzen & Hall, 1982; Itakura et al., 1977) using available codon usage tables (SEQ ID No. 3 was more adapted towards expression in cotton, SEQ ID No. 5 more towards corn), and also to eliminate stretches of AT or GC nucleotides longer then 5 or 6, preferably longer then 5, nucleotides, and also to insert suitable restriction sites.

Also, the N-terminus of a Cry protein can be modified to have an optimum translation initiation context, thereby adding or deleting one or more amino acids at the N-terminal end of the protein. In most cases, it is preferred that the proteins of the invention to be expressed in plants cells start with a Met-Asp or Met-Ala dipeptide for optimal translation initiation, requiring the insertion in the *cry2A* DNA of a codon encoding an Asp or Ala amino acid downstream of the start codon as a new second codon.

Of course, any DNA sequence differing in its codon usage but encoding the same protein or a similar protein with substantially the same insecticidal activity, can be constructed, depending on the particular purpose. It has been described in prokaryotic and eucaryotic expression systems that changing the codon usage to that of the host cell is desired for gene expression in foreign hosts (Bennetzen & Hall, 1982: ltakura et al., 1977). Furthermore, *Bt* crystal protein genes are known to have no bias towards eucaryotic codons, and to be very AT-rich (Adang et al., 1985, Schnepf et al., 1985). Codon usage tables are available in the literature (Wada et al., 1990; Murray et al., 1989) and in the major DNA sequence databases (e.g. EMBL at Heidelberg. Germany). Accordingly, synthetic DNA sequences can be constructed so that the same or substantially the same proteins are produced, It is evident that several DNA sequences can be made once the amino acid sequence of the Cry proteins of this invention is known. Such other DNA sequences include synthetic or semi-synthetic DNA sequences that have been changed in order to inactivate certain sites in the gene, e.g. by selectively inactivating certain cryptic regulatory or processing elements present In the native sequence as described In PCT publications WO 91/16432 and WO 93/09218. or by adapting the overall codon usage to that of a more related host organism preferably that of the host organism in which expression is desired. Several techniques for modifying the codon usage to that preferred by the host cells can be found in patent and scientific literature. The exact method of codon usage modification is not critical for this invention as long as most or all of the cryptic regulatory sequences or processing elements have been replaced by other sequences. Examples of DNA sequences optimized for expression in plants are shown in enclosed SEQ ID Nos. 3 and 5.

Small modifications to a DNA sequence such as described above can be routinely made, i.e.. by PCR-mediated mutagenesis (Ho et al.,1989, White et al., 1989).

More profound modifications to a DNA sequence can be routinely done by de novo DNA synthesis of a desired coding region using available techniques.

With the term "substantially the same", when referring to the amino acid sequence of a Cry protein, is meant to include an amino acid sequence that differs in no more than 5 %, preferably no more than 2 %, to the amino acid sequence of the protein compared to; and when referring to toxicity of Cry protein, is meant to include a protein whose LC₅₀ value obtained under the same conditions of bioassay differs by no more then 10 %, preferably no more than 5 %, of the LC₅₀ value obtained for the protein compared to.

The term "domain" of a Cry toxin as used herein means any part(s) or domain(s) of the toxin with a specific structure that can be transferred to another (Cry) protein for providing a new hybrid protein with at least one functional characteristic (e.g., the binding and/or toxicity characteristics) of the Cry toxin of the invention (Ge et al., 1991). Such parts can form an essential feature of the hybrid Bt protein with the binding and/or toxicity characteristics of the Cry protein of this invention. Such a hybrid protein can have an enlarged host range, an improved toxicity and/or can be used in a strategy to prevent insect resistance development (European Patent Publication ("EP") 408 403; Visser et al., 1993).

The cry DNA sequences of the invention, prepared from total DNA, can be ligated in suitable expression vectors and transformed in *E. coli*, and the clones can then be screened by conventional colony immunoprobing methods (French et al., 1986) for expression of the toxin with monoclonal or polyclonal antibodies raised against the Cry proteins.

Also, the *cry* DNA of the invention, can be ligated in suitable *Bt* shuttle vectors (Lereclus et al., 1992) and transformed in a crystal minus *Bt*-mutant. The clones can then be screened for production of crystals (detected by microscopy) or crystal proteins (detected by SDS-PAGE), or can be tested for their insecticidal activity compared to the control crystal-minus strain.

The genes encoding the Cry proteins of this invention can be sequenced in a conventional manner (Maxam and Gilbert, 1980; Sanger, 1977) to obtain the DNA sequence. Sequence comparisons indicated that the genes are different from previously described genes encoding protoxins and toxins with activity against Lepidoptera (see, e.g., Höfte and Whiteley, 1989; Crickmore, et al., 1998; and the October 16, 2000 update on the *Bt* nomenclature website corresponding to the Crickmore et al. (1998) publication, found at
http://epunix.biols.susx.ac.uk/Home/Neil Crickmore/Bt/index.html). Also, the Cry2A proteins of the invention are novel over any of the Bacillus thuringiensis crystal protein sequences in the December 13,2001 update of this Bt nomenclature website.

An insecticidally effective part of the DNA sequences, encoding an insecticidally effective portion of the newly identified Cry protein protoxin forms, can be made In a conventional manner after sequence analysis of the gene. In such fragments, it is preferred that at least the sequence homologous to the conserved sequence block 5 of *Bt* crystal proteins (Hofte & Whiteley, 1989; Schnepf et al., 1998) is included in such protein, preferably up to two amino acids after this homologous region. For the Cry2Ae proteins, this homologous region ends at amino acid position 625 in SEQ ID Nos. 2. The amino acid sequence of the Cry proteins can be determined from the DNA sequence of the isolated DNA sequences. By "an insecticidally effective part (or portion or fragment)" of DNA sequences encoding the Cry protein, also referred to herein as "truncated gene" or "truncated DNA", is meant a DNA sequence encoding a polypeptide which has fewer amino acids than the Cry protein protoxin form but which is insecticidal.

In order to express all or an insecticidally effective part of the DNA sequence encoding a Cry protein of this invention in *E. coli*, in other *Bt* strains and in plants, suitable restriction sites can be introduced, flanking the DNA sequence. This can be done by site-directed mutagenesis, using well-known procedures (Stanssens et al., 1989; White et al., 1989). In order to obtain improved expression in plants, the codon usage of the *cry* gene or insecticidally effective *cry* gene part of this invention can be modified to form an equivalent, modified or artificial gene or gene part in accordance with PCT publications WO 91/16432 and WO 93/09218; EP 0 358 962 and EP 0 359 472, or the *Bt* genes or gene parts can be inserted in the plastid, mitochondrial or chloroplast genome and expressed there using a suitable promoter (e.g., Mc Bride et al., 1995; US patent 5,693,507). For obtaining enhanced expression in monocot plants such as corn, an intron, preferably a monocot intron, also can be added to the chimeric gene, and the DNA sequence of the *cry* gene or its insecticidal part can be further changed in a translationally neutral manner, to modify possibly inhibiting DNA sequences present in the gene part by means of site-directed intron insertion and/or by introducing changes to the codon usage, e.g., adapting the codon usage to that most preferred by plants, preferably the specific relevant plant genus, (Murray et al., 1989) without changing significantly, preferably without changing, the encoded amino acid sequence.

In accordance with one embodiment of this invention, it is preferred that the proteins are targeted to intracellular organelles such as plastids, preferable chloroplasts, mitochondria, or are secreted from the cell, potentially optimizing protein stability and/or expression. For this purpose, the chimeric genes of the invention comprise a coding region encoding a signal or target peptide, linked to the Cry protein coding region of the invention. Particularly preferred peptides to be included in the proteins of this invention are the transit peptides for chloroplast or other plastid targeting, especially duplicated transit peptide regions from plant genes whose gene product is targeted to the plastids, the optimized transit peptide of Capellades et al. (US Patent 5,635,618), the transit peptide of ferredoxin-NADP⁺oxidoreductase from spinach (Oelmuller et al., 1993), the transit peptide described in Wong et al. (1992) and the targeting peptides in published PCT patent application WO 00/26371. Also preferred are peptides signalling secretion of a protein linked to such peptide outside the cell, such as the secretion signal of the potato proteinase inhibitor II (Keil et al., 1986), the secretion signal of the alpha-amylase 3 gene of rice (Sutliff et al., 1991) and the secretion signal of tobacco PR1 protein (Cornelissen et al., 1986).

Particularly useful signal peptides in accordance with the invention include the chloroplast transit peptide (e.g., Van Den Broeck et al. (1985), or the optimized chloroplast transit peptide of US patent 5, 510,471 and US patent 5,635,618 causing transport of the protein to the chloroplasts, a secretory signal peptide or a peptide targeting the protein to other plastids, mitochondria, the ER, or another organelle. Signal sequences for targeting to intracellular organelles or for secretion outside the plant cell or to the cell wall are found in naturally targeted or secreted proteins, preferably those described by Klösgen et al. (1989), Klösgen and Weil (1991). Neuhaus & Rogers (1998), Bih et al. (1999), Morris et al. (1999), Hesse et al. (1989), Tavladoraki et al. (1998), Terashima et al. (1999), Park et al. (1997), Shcherban et al. (1995), particularly the signal peptide sequences from targeted or secreted proteins of corn, cotton, rice or soybean.

Furthermore, the binding properties of the Cry proteins of the invention can be evaluated, using methods known in the art (e.g., Van Rie et al., 1990), to determined if the Cry proteins of the invention bind to sites on the insect midgut that are not recognized (or competed for) by other, known Cry or other *Bt* proteins. *Bt* toxins with different binding sites for which there is non-competitive binding in relevant susceptible insects are very valuable to replace known *Bt* toxins to which insects may have developed resistance, or to use in combination with *Bt* toxins having a different mode of action to prevent or delay the development of insect resistance against *Bt* toxins, particularly when expressed in a plant. Because of the characteristics of the newly isolated *Bt* toxins, they are extremely useful for transforming plants, e.g. monocots such as corn or rice and dicots such as cotton, soybean and Brassica species plants, to protect these plants from insect damage. It has been described that in Helicoverpa zea, the Cry2Aa protein does not share binding sites with the Cry1Ac protein (English et al., 1994). Similarly, it is expected that the binding properties of the Cry2A proteins of the current invention will be different compared to those of Cry1 or Cry9 toxins currently used in transgenic plants in the relevant insect pests. Such different binding properties can be measured by routine binding assays as described above. Especially for insect resistance management purposes for a specific insect pest, it is preferred to combine a Cry2Ae protein of this invenion with another insect control protein, particularly a Bt crystal protein, which does not recognize at least one binding site recognized by such Cry2Ae protein. Preferred insect control proteins to combine with the Cry2Ae protein, particularly for simultaneous expression in plants, preferably cotton plants, include the Cry1F protein or hybrids derived from a Cry1F protein (e.g., the hybrid Cry1A-Cry1F proteins described in US patents 6,326,169; 6,281,016; 6,218,188, or toxic fragments thereof), the Cry1A-type proteins or toxic fragments thereof, preferably the Cry1Ac protein or hybrids derived from the Cry1Ac protein (e.g., the hybrid Cry1Ab-Cry1Ac protein described in US patent 5,880,275), the VIP3Aa protein or a toxic fragment thereof as descibed in Estruch et al., 1996 and US Patent 6,291,156, insecticidal proteins from Xhenorhabdus, Serratia or Photorhabdus species strains (e.g., Waterfield et al., 2001; ffrench-Constant and Bowen, 2000). In one embodiment, such co-expression is easily obtained by transforming a plant already expressing an insect control protein with a Cry2Ae of this invention, or by crossing plants transformed with the insect control protein and plants transformed with the Cry2Ae protein of this invention. For cotton plants, preferably the Cry2Ae protein is used as first insect control protein and as second insect control protein the Cry1Ac or VIP3Aa proteins or derivatives thereof are used. Methods for obtaining expression of different Bt (or similarly, for other insect control proteins) insecticidal proteins in the same plant in an effort to minimize or prevent resistance development to transgenic insect-resistant plants are described in EP patent 0 408 403.
The Cry2Ae proteins of this invention can also conveniently be used to control insects in case insect resistance develops against insect control proteins, such as the Cry1 Bt proteins, which are currently already commercialized in transgenic plants.

Preferably, for selection purposes but, also for increasing the weed control options, the transgenic plants of the invention are also transformed with a DNA encoding a protein conferring resistance to a broad-spectrum herbicide, e.g., herbicides based on glufosinate or glyphosate.

The insecticidally effective *cry* gene part or its equivalent, preferably the *cry* chimeric gene, encoding an insecticidally effective portion of the Cry protoxin, can be stably inserted in a conventional manner into the nuclear genome of a single plant cell, and the so-transformed plant cell can be used in a conventional manner to produce a transformed plant that is insect-resistant. In this regard, a disarmed Ti-plasmid, containing the insecticidally effective cry gene part, in *Agrobacterium* tumefaciens can be used to transform the plant cell, and thereafter, a transformed plant can be regenerated from the transformed plant cell using the procedures described, for example, in EP 0 116 718, EP 0 270 822. PCT publication WO 84/02913 and published European Patent application ("EP") 0 242 246 and In Gould et al. (1991). Preferred Ti-plasmid vectors each contain the insecticidally effective *cry* gene part between the border sequences, or at least located to the left of the right border sequence, of the T-DNA of the Ti-plasmid. Of course, other types of vectors can be used to transform the plant cell, using procedures such as direct gene transfer (as described, for example In EP 0 233 247), pollen mediated transformation (as described, for example In EP 0 270 358. PCT publication WO 85/01856, and US Patent 4,684,611), plant RNA virus-mediated transformation (as described, for example in EP 0 067 553 and US Patent 4,407,956), liposome-mediated transformation (as described, for example in US Patent 4,536,475), and other methods such as the recently described methods for transforming certain lines of corn (e.g., US patent 6,140.663; Fromm et al., 1990; Gordon-Kamm et al., 1990) and rice (Shimamoto et al., 1989; Datta et al., 1990) and the method for transforming monocots generally (PCT publication WO 92/09696). For cotton transformation, especially preferred is the method described in PCT patent publication WO 00/71733. For soybean transformation, reference is made to methods known in the art, e.g., Hinchee et al. (1988) and Christou et al. (1990) or the method of WO 00/42207.

Also, besides transformation of the nuclear genome, also transformation of the plastid genome, preferably chloroplast genome, is included in the invention. Kota et al. (1999) have described a method to overexpress a Cry2Aa protein in tobacco chloroplasts.

The resulting transformed plant can be used in a conventional plant breeding scheme to produce more transformed plants with the same characteristics or to introduce the insecticidally effective *cry* gene part in other varieties of the same or related plant species. Seeds, which are obtained from the transformed plants, contain the insecticidally effective *cry* gene part as a stable genomic insert. Cells of the transformed plant can be cultured in a conventional manner to produce the insecticidally effective portion of the Cry protoxin, preferably the Cry toxin, which can be recovered for use in conventional insecticide compositions against Lepidoptera (US Patent 5,254,799).

The insecticidally effective *cry* gene part, preferably the truncated *cry* gene, is inserted in a plant cell genome so that the inserted gene is downstream (i.e., 3') of, and under the control of, a promoter which can direct the expression of the gene part in the plant cell. This is preferably accomplished by inserting the *cry* chimeric gene in the plant cell genome, particularly in the nuclear or plastid (e.g., chloroplast) genome. Preferred promoters include: the strong constitutive 35S promoters (the "35S promoters") of the cauliflower mosaic virus (CaMV) of isolates CM 1841 (Gardner at al., 1981), CabbB-S (Franck et al., 1980) and CabbB-JI (Hull and Howell, 1987); the 35S promoter described by Odell et al. (1985), promoters from the ubiquitin family (e.g., the maize ubiquitin promoter of Christensen et al., 1992, see also Comejo et al., 1993), the gos2 promoter (de Pater et al.. 1992), the emu promoter (Last et al., 1990), Arabidopsis actin promoters such as the promoter described by An et al. (1996), rice actin promoters such as the promoter described by Zhang et al. (1991); promoters of the Cassava vein mosaic virus (WO 97/48819, Verdaguer et al. (1998)), the pPLEX series of promoters from. Subterranean C!over Stunt Virus (WO 96/06932. particularly the S7 promoter), a alcohol dehydrogenase promoter, e.g., pAdh1S (GenBank accession numbers X04049, X00581), and the TR1' promoter and the TR2' promoter (the "TR1' promoter" and "TR2' promoter", respectively) which drive the expression of the 1' and 2' genes, respectively, of the T-DNA (Velten et al., 1984). Alternatively, a promoter can be utilized which is not constitutive but rather is specific for one or more tissues or organs of the plant (e.g., leaves and/or roots) whereby the inserted cry gene part is expressed only in cells of the specific tissue(s) or organ(s). For example, the insecticidally effective *cry* gene part could be selectively expressed in the leaves of a plant (e.g., corn, cotton) by placing the insecticidally effective gene part under the control of a light-inducible promoter such as the promoter of the ribulose-1,5-bisphosphate carboxylase small subunit gene of the plant itself or of another plant such as pea as disclosed in US Patent 5,254,799. Another alternative is to use a promoter whose expression is inducible, preferably by wounding such as insect feeding, e.g., the MPI promoter described by Cordera et al. (1994), or by chemical factors.

The insecticidally effective cry gene part Is inserted in the plant genome so that the inserted gene part is upstream (i.e., 5') of suitable 3' end transcription regulation signals (i.e., transcript formation and polyadenylation signals). This is preferably accomplished by inserting the *cry* chimeric gene in the plant cell genome. Preferred polyadenylation and transcript formation signals include those of the noplaine synthase gene (Depicker et al.,1982), the octopine synthase gene (Gielen et al., 1984) and the T-DNA gene 7 (Velten and Schell, 1985), which act as 3'-untranslated DNA sequences in transformed plant cells.

The insecticidally effective *cry* gene part can optionally be inserted in the plant genome as a hybrid gene (US Patent 5.254,789; Vaeck et al., 1987) under the control of the same promoter as a selectable or scorable marker gene, such as the *neo* gene (EP 0 242 236) encoding kanamycin resistance, so that the plant expresses a fusion protein which is easily detectable.

Transformation of plant cells can also be used to produce the proteins of the invention in large amounts in plant cell cultures, e.g., to produce a Cry2A protein that can then be applied onto crops after proper formulation. When reference to a transgenic plant cell is made herein, this refers to a plant cell (or also a plant protoplast) as such in isolation or in tissue culture, or to a plant cell (or protoplast) contained in a plant or in a differentiated organ or tissue, and both possibilities are specifically included herein. Hence, a reference to a plant cell in the description or claims is not meant to refer only to isolated cells in culture, but refers to any plant cell, wherever it may be located or In whatever type of plant tissue or organ it may be present.

All or part of the *cry* gene, encoding an anti-lepidopteran protein, can also be used to transform other bacteria, such as a *B. thuringiensis* which has insecticidal activity against Lepidoptera or Coleoptera. Thereby, a transformed *Bt* strain can be produced which is useful for combatting a wide spectrum of lepidopteran and coleopteran insect pests or for combatting additional lepidopteran insect pests. Transformation of bacteria, such as bacteria of the genus *Pseudomonas, Agrobacterium, Bacillus* or *Escherichia,* with all or part of the *cry* gene of this invention, incorporated in a suitable cloning vehicle, can be carried out in a conventional manner, preferably using conventional electroporation techniques as described in Mahillon et al. (1989) and in PCT Patent publication WO 90/06999.

Transformed Bacillus species strains containing the cry gene of this invention can be fermented by conventional methods (Dulmage,1981; Bernhard and Utz, 1993) to provide high yields of cells. Under appropriate conditions which are well understood (Dulmage, 1981), these strains each sporulate to produce crystal proteins containing the Cry protoxin in high yields.

An insecticidal, particularly anti-lepidopteran, composition of this invention can be formulated in a conventional manner using the microorganisms transformed with the *cry* gene, or preferably their respective Cry proteins or the Cry protoxin, toxin or insecticidally effective protoxin portion as an active ingredient, together with suitable carriers, diluents, emulsifiers and/or dispersants (e.g., as described by Bernhard and Utz, 1993). This insecticide composition can be formulated as a wettable powder, pellets, granules or dust or as a liquid formulation with aqueous or non-aqueous solvents as a foam, gel, suspension, concentrate, etc.

A method for controlling insects, particularly Lepidoptera, in accordance with this invention can comprise applying (e.g., spraying), to a locus (area) to be protected, an insecticidal amount of the Cry proteins or host cells transformed with the *cry* gene of this invention. The locus to be protected can include, for example, the habitat of the insect pests or growing vegetation or an area where vegetation is to be grown.

This invention further relates to a method for controlling lepidopteran soybean insect pests, particularly Lepidopteran rice stemborers, rice skippers, rice cutworms, rice armyworms, rice caseworms or rice leaffolders, preferably an insect selected from the group consisting of: Chilo suppressalis, Chilo partellus, Scirpophaga incertulas, Sesamia inferens, Cnaphatocrocis medinalis. Marasmia patnalis, Marasmia exigua, Marasmia ruralis, Scirpophaga innotata, which method comprises applying to an area or plant to be protected, a Cry2Ae protein as defined herein, (i.e., by planting a rice plant transformed with a *cry2Ae* gene of this invention, or spraying a composition containing a Cry2Ae protein of this invention). The invention also relates to the use of the Cry2Ae protein, against Lepidopteran rice insect pests to minimize damage to rice plants.

This invention further relates to a method for controlling lepidopteran cotton insect pests, which method comprises applying to an area or plant to be protected, a a Cry2Ae protein as defined herein, (i.e.. by planting a rice plant transformed with a cry2Ae gene of this invention, or spraying a composition containing a Cry2Ae protein of this invention). The invention also relates to the use of the Cry2Ae protein, against Lepidopteran rice insect pests to minimize damage to rice plants.

This invention also relates to a method for controlling lepidopteran rice insect pests, particularity Lepidopteran rice stemborers, rice skippers, rice cutworms, rice armyworms, rice caseworms or rice leaffolders, preferably an insect selected from the group consisting of: Chilo suppressalis, Chilo partellus, Scirpophaga incertulas, Sesamia inferens, Cnaphalocrocis medinalis, Marasmia patnalis, Marasmia exigua, Marasmia ruralis, Scirpophaga innotata, which method comprises applying to an area or plant to be protected, a Cry2Ae protein as defined herein, (i.e., by planting a rice plant transformed with a *cry2Ae* gene of this invention, or spraying a composition containing a Cry2Ae protein of this invention). The invention also relates to the use of the Cry2Ae protein, against Lepidopteran rice insect pests to minimize damage to rice plants.

To obtain the Cry protoxin or toxin, cells of the recombinant hosts expressing the Cry protein can be grown in a conventional manner on a suitable culture medium and then lysed using conventional means such as enzymatic degradation or detergents or the like. The protoxin can then be separated and purified by standard techniques such as chromatography, extraction, electrophoresis, or the like. The toxin can then be obtained by trypsin digestion of the protoxin.

These and/or other embodiments of this invention are reflected in the wordings of the claims, that form part of the description of the invention.

The following Examples illustrate the invention, and are not provided to limit the invention or the protection sought. The sequence listing referred to in the Examples, the Claims and the Description is as follows:

### Sequence Listing:

SEQ ID No. 1 - amino acid and DNA sequence of Cry2Ae protein and DNA
SEQ ID No. 2 - amino acid sequence of Cry2Ae protein.
SEQ ID No. 3 - artificial *cry2A*e DNA sequence for expression in cotton.
SEQ ID No. 4 - amino acid sequence of Cry2Ae protein encoded by the DNA of SEQ ID No. 3.
SEQ ID No. 5 - artificial *cry2Ae* DNA sequence for expression in com.

Unless otherwise stated in the Examples, all procedures for making and manipulating recombinant DNA are carried out by the standard procedures described in Sambrook et al., Molecular Cloning - A Laboratory Manual, Second Ed., Cold Spring Harbor Laboratory Press, NY (1989), and in Volumes 1 and 2 of Ausubel et al. (1994) Current Protocols In Molecular Biology. Current Protocols, USA. Standard materials and methods for plant molecular biology work are described in Plant Molecular Biology Labfax (1993) by R.R.D. Croy, jointly published by BIOS Scientific Publications Ltd (UK) and Blackwell Scientific Publications (UK). Procedures for PCR technology can be found in "PCR protocols: a guide to methods and applications". Edited by M.A. Innis, D.H. Gelfand, J.J. Sninsky and T.J. White (Academic Press. Inc., 1990).

### EXAMPLES

### Example 1: Characterization of the strains.

The BTS02761A and BTS01099E strains where isolated from grain dust collected in the Philippines (South Tagalog) and Belgium (Deerlijk), respectively.

Each strain can be cultivated on conventional standard media, preferably T₃ medium (tryptone 3 g/l, tryptose 2 g/l, yeast extract 1.5 g/l, 5 mg MnCl₂, 0.05 M Na₂HPO₄.2H₂O, 0.05 M NaH₂PO₄.H₂O, pH 6.8 and 1.5% agar), preferably at 28 °C. For long term storage, it is preferred to mix an equal volume of a spore-crystal suspension with an equal volume of 50% glycerol and store this at -70 °C or lyophilize a spore-crystal suspension. For sporulation, growth on T₃ medium is preferred for 72 hours at 28°C, followed by storage at 4°C. The crystal proteins produced by the strains during sporulation are packaged in crystals.

### Example 2 : Insecticidal activity of the BTS02761A and BTS01099E strains against selected lepidopteran insect species.

Toxicity assays were performed on neonate larvae of *Helicoverpa* zea, *Holicoverpa armigera, Heliothis virescens*, *Ostrinia nubilalis, Spodoptera frugiperda,* and *Sesamia nonagrioides* fed on an artificial diet layered with undiluted alcaline (pH12) extract of spore-crystal mixtures from either BTS01099E or BTS02761A.
The artificial diet (Vanderzant, 1962) was dispensed in wells of Costar 48-well plates. 25 microliter of the extract on the surface of the diet and dried in a laminar air flow. One larva was placed In each well and 18 larvae were used per sample. Dead and living larvae were counted on the seventh day. The percentage of dead larvae are shown in Table I below.

Mixtures of spore/crystals from each of the strains BTS02761A and BTS01099E were tested in bioassays and gave the following results:

**Table I:**

| Strain | Mortality (%) | | | | |
|---|---|---|---|---|---|
| | Hz | Hv | Sf | On | Sn |
| BTS02761A | 17* | 94 | 5 | 88 | 77 |
| BTS01099E | 70 | 100 | NT | 90 | NT |

| | | | | | |
|---|---|---|---|---|---|
| *: surviving larvae slightly affected in their growth Negative controls (standard diet): Hz 6% M. Hv:17% M, Sf: 0% M. Hz: Helicoverpa zea; Hv: Heliothis virescens; Sf: Spodoptera frugiperda; On: Ostrinia nubilalis; Sn: Sesamia nonagroides (NT means not tested). | | | | | |

### Example 3 : Identification and characterization of new cry2A genes from Bt strains BTS01099E and BTS02761A.

Using appropriate primers, a portion of the *cry2A* gene(s) from the BTS02761A and BTS01099E strains were amplified; subsequently these amplification products were digested with restriction enzymes. The pattern obtained was then compared with the pattern that is obtained when such digests are performed on amplification products derived from strains containing known *cry2A* genes. Based on the restriction digest pattern, the *cry2A* genes from strains BTS02761A and BTS01099E appeared to be novel. Therefore, the amplification product was sequenced. This confirmed that the amplified fragments were derived from novel *cry2A* genes: strain BTS02761A contained a novel *cry2A*-like gene, whereas strain 1099E contained two novel *cry2A*-like genes.

Total DNA from strains BTS02761A and BTS01099E was treated with Sau3A, size fractionated and fragments of 7 to 10 kb were ligated into pUC19I (a derivative of pUC19), cut with BamHI and treated with TsAP (heat stable alkaline phospatase). This ligation mixture was electroporated in E. coli XL1 Blue.

Colony hybridization, using the DIG-labeled PCR fragments as probes, identified positive clones. The recombinant E. coil strains were deposited on October 6, 2000 at the Vakgroep voor Moleculaire Biologie-Plasmidencollectie, Universiteit Gent, K.L. Ledeganckstraat 35, B-9000 Gent, Belgium (hereinafter abbreviated as "BCCM-LMBP*) under the following accession numbers: BCCM-LMBP 4247 for strain XL1Blue:pUC1099E/cry2clone1, which encodes a protein named Cry2Af; BCCM-LMBP 4248 for strain XL1Blue:pUC1099E/cry2clone7, which encodes a protein named Cry2Ae; and BCCM-LMBP 4249 for strain XL1Blue:pUC2761A/cry2clone141, which encodes a protein named Cry2Ag. The genes can be isolated from these deposited clones by a NotI-FseI digest. The insert from these clones was subcloned into shuttle vector pSL40I. The resulting plasmid was first transformed into E. coli GM2163. A plasmid prep from this strain was then electroporated into a crystal-minus B. thuringiensis variety berliner 1715 strain.
An alkaline extract prepared from a spore/crystal mixture from the recombinant Bt strains was then used in bioassays to evaluate the toxicity of the novel Cry2A proteins. This extract was tested in the assay as described above in Example 1. The results are shown in Table II:

**Table II:**

| Toxin | Conc. | Mortality (%) | | | | | |
|---|---|---|---|---|---|---|---|
| | | Ha | Sf | On | Sn | Hz | Hv |
| Cry2Ae | 1930 | 83 | 44 | NT | 100 | 100 | NT |
| Cry2Ag | 1160 | 0 | 0 | 78 | 50 | 29 | 100 |
| Cry2Aa | 470 | 61 | 55 | 50 | 94 | 95 | 100 |

| | | | | | | | |
|---|---|---|---|---|---|---|---|
| "Conc.": total protein concentration of strain extract using the Bradford method (microgr/ml); "Ha": *Heliothis armigera*, the other abbreviations are as used above in Table I; the included controls (normal diet, PBS-BSA addition or unstransformed crystal-minus Bt strain 1715) give no significant mortality. | | | | | | | |

Also, the recombinant clone expressing the Cry2Af protein shows a significant mortality when tested on selected Lepidopteran insects.

Also, an analysis was done to determine the LC50 and LC90 values for the recombinantly produced Cry2Ae protein, in comparison with the known Cry2Aa and Cry2Ab proteins.
For this assay, insect-specific artificial diet was dispensed in wells of Costar 24-well plates. 50 microliter of alcaline (pH12) extract of spore-crystal mixtures of the recombinant Bt strain containing the cry2Ae gene originating from XL1 Blue:pUC1099Eclone7; was applied on the surface of the diet and dried in a laminar air flow. The diet for *S. frugiperda* en *O. nubilalis* contained: 1000ml water; agar: 20 g; cornflour: 112 g; wheat germ: 28 g; yeast: 30 g; ascorbic acid: 4.8 g; benzoic acid: 1.2 g; nipagin:1 g; aureomycin: 0.06 g; nystatin: 0.03 g. The diet for H. virescens en H. zea contained: 1000ml water; agar: 20 g; soyflour: 81 g; wheat germ: 36 g, sucrose: 14.7 g; corn oil: 5 ml; Wesson salt mixture: 10 g; Vanderzant vitamin mixture: 9.5 g; sorbic acid; 1.1g; nipagin: 1 g: aureomycin: 0.34 g; nystatin: 0.06 g. Different protein concentrations were tested so that an LC50 value could be determined. For tests on *H. zea*, *H. virescens* and *S. frugiperda,* one larva was placed in each well and 20 larvae were used per sample. For tests on O. nubilalis, two larva were placed in each well and 24 larvae were used per sample. Dead and living larvae were counted on the seventh day (on the sixth day for S. frugiperda, on the fifth day for O. nubilalls). The LC50 and LC90 values were calculated with probit analysis (POLO program, LeOra Software, 1987, POLO-PC. A user's guide to probit or logic analysis.Berkeley, California). The results are shown in Table III below.

**Table III:**

| Toxin | Conc. | LC50(LC90) values, both in ng/cm² | | | |
|---|---|---|---|---|---|
| | | Sf | Hz | Hv | On |
| Cy2Ae | 1160 | 1154 | 62 | 10 | *188 |
| | (*1930) | (3708) | (655) | (20) | (*1383) |
| Cry2Aa | 2910 | 2906 | 1921 | 35 | *294 |
| | (*470) | (10945) | (7740) | (138) | (*2854) |
| Cry2Ab | 1290 | 1498 | 448 | 82 | NT |
| | | (8150) | (2152) | (248) | |

| | | | | | |
|---|---|---|---|---|---|
| NT: not tested; Conc.: total protein concentration in alcaline extract of recombinant Bt strain producing the relevant protein in microgr/ml; an asterisk denotes that the result for O. nubilalis was obtained with a different batch having a different protein concentration (indicated between brackets under the column "Conc."); controls (normal diet, added PBS-BSA or crystal-minus control Bt strain) give no more then 0-5% mortality. | | | | | |

Using the same experimental setup as above for Ostrinia nubilalis, but using purified Cry2Ae protein against the velvetbean caterpillar, *Anticarsia gemmata*/*is,* (testing 20 wells with 1 larva per concentration) a high activity of this protein against this important soybean pest insect was found. The LC₅₀ value for the purified Cry2Ae protein to this insects was found to be 0.44 ng/cm² (at 95% confidence level; this LC₅₀ value is the mean value of 2 assays of different bio-batches of purified protein), the LC₉₀ value was found to be 7.79 ng/cm² (at the 95 % confidence level; this LC₉₀ value is the mean value of 2 bio-assays of different batches of purified protein). Using the same experimental setup as above for Ostrinia with purified Cry2Ae protein, the significant toxicity of this protein to Helicoverpa Zea and Ostrinia Nubilalis was confirmed (LC₅₀ values to these insects were found to be 145.1 and 48.31 ng/ cm², respectively (at 95 % confidence level, these LC₅₀ values are the mean values of 2 bio-assays of different batches of purified protein on each respective insect)).

These results show that the new Cry proteins of the invention, and particularly the Cry2Ae protein, are useful proteins with high activity to relevant Lepidopteran Insect pests, particularly to Heliothis zea, Ostrinia nubilalis, Anticarsia gemmatalis, and Helicoverpa zea which are commercially damaging insect pests for plants such as soybean, cotton and com.

The sequences determined for the isolated *cry2A* genes of the invention, and the determined amino acid sequence, are shown in the enclosed Sequence Listing. Pairwise alignments using the GAP program in the Wisconsin package of GCG indicated the levels of sequence identity with other Cry2A sequences (for the sequences of the known Cry2A proteins and DNAs, see Crickmore et al. (1998) and the above recited internet website), as shown in Table IVA and IVB (GCG defaults were used within the GAP program; for the amino acid sequence comparisons, the blosum62 scoring matrix was used, for the DNA sequence comparisons, the nwsgapdna scoring matrix was used).

**Table IV.A. Percentage sequence identity at the protein level:**

| | Cry2Ae1 | CryZAf1 | Cry2Ag1 |
|---|---|---|---|
| Cry2Aa1 | 90.837 | 88.942 | 78.905 |
| Cry2Ab1 | 89.889 | 94.471 | 77.331 |
| Cry2Ac1 | 80.547 | 80.388 | 79.869 |
| Cry2Ad1 | 87.362 | 91.943 | 76.849 |
| Cry2Ae1 | | 93.365 | 79.871 |
| Cry2Af1 | | | 79.549 |

**Table IV.B. Percentage sequence identity at the DNA level:**

| | *cry2Ae1* | *cry2Af1* | *cry2Ag1* |
|---|---|---|---|
| *cry2Aa1* | 91.206 | 89.995 | 81.994 |
| *cry2Ab1* | 91.890 | 94.839 | 81.404 |
| *cry2Ac1* | 84.298 | 85.209 | 84.041 |
| *cry2Ad1* | 90.627 | 93.470 | 81.136 |
| *cry2Ae1* | | 94.578 | 81.589 |
| *cry2Af1* | | | 82.233 |

### Example 4: production of the novel Cry proteins in transformed plants.

Chimeric genes each encoding the Cry2Ae, Cry2Af and Cry2Ag proteins are made using well known procedures, using promoters such as the CaMV 35S (Hull and Howell, 1987) and ubiquitin (Christensen et al., 1992) promoters- Preferably, the codon usage of the open reading frame is adapted to that of the host plant so as to optimize expression efficiency, as described in published PCT patent application WO 94/12264. Also, in some chimeric genes DNA sequences encoding a transit peptide (as described in the description) are included to target the Cry2A protein of the invention to the plant chloroplasts.
For transformation of corn and cotton with a chimeric gene encoding the Cry2Ae protein, several chimeric gene constructs were inserted in Agrobacterium strain plasmids. These constructs included: constructs pACS9 and pacts11 wherein the *cry2Ae* coding sequence of SEQ ID No. 3 was functionally linked to the 35S2 promoter from Cauliflower Mosaic Virus (Odell et al., 1985), a leader sequence from the chlorophyl a/b binding protein gene from Petunia (Harpster et al., 1988), and a 3' transcript termination and polyadenylation region of the 35S gene from Cauliflower Mosaic Virus (Sanfacon et al, 1991), and constructs pACS12 and pACS13 with the same regulatory regions and the same *cry2Ae* coding region, except that also a DNA sequence encoding the TpssuAt transit peptide allowing chloroplast targeting (Krebbers et al., 1988) was inserted at the 5' end of the *cry2Ae* coding region, so that a transit peptide fusion protein is produced. These constructs also included either a DNA sequence encoding a glyphosate herbicide resistance protein (described in published PCT patent application WO 97/04103. linked to an optimized transit peptide (US patent 5,635,618)) or a DNA sequence encoding a glufosinate herbicide resistance protein (Thompson et al., 1987) as selectable marker under the control of the CsVMV promoter of the Cassava Vein Mosaic Virus (Verdaguer et al., 1996, 1998) and the 3' transcript termination and polyadenylation region of the nopaline synthase gene (Depicker et al., 1982).
Corn cells were stably transformed with the pACS9, pACS11. pACS12 and pACS13 constructs by either Agrobacterium-mediated transformation as described in US Patent 6,140,553. Cotton cells were stably transformed with the pACS 9, pACS11, pACS12 and pACS13 constructs using the transformation method described in PCT patent publication WO 00/71733. Rice cells are stably transformed with the method described in published PCT Rice cells are stably transformed with the method described in published PCT patent application WO 92/09696. Tobacco cells were stably transformed with the pACS11 and pACS12 constructs using Agrobacterium-mediated transformation, essentially as described in EP patent 0 116 718 or Deblaere et al. (1987).
The transformed cells and plantlets regenerated therefrom are grown in media containing the selective agents phosphinotricin or glyphosate, so that most if not all of the regenerated plants will be transformed.
Regenerated transformed tobacco, corn, cotton and rice plants are selected by Cry2A ELISA, Northern and Southern blot and according to insecticidal efficacy and agronomic characteristics. Chimeric *cry2A* gene-containing progeny plants show improved resistance to insects compared to untransformed control plants with an appropriate segregation of the insect resistant and the transformed phenotype. Protein and RNA measurements show that plants with increased insect resistance have a higher expression of the novel Cry2A protein in their cells.

### REFERENCES CITED

Adang et al.(1986). Gene 36, 289.
An et al. (1996). Plant J. 10. 107.
Bennetzen & Hall.(1982).J. Biol. Chem. 257,3026-3031.
Berhard, K, and Utz, R., "Production of Bacillus thuringiensis insecticides for experimental and commercial uses", In Bacillus thuringiensis, An Environmental Biopesticide: Theory and Practice, pp.255-267, eds. Entwistle, P.F., Cory, J.S., Bailey, M.J. and Higgs, S., John Wiley and Sons, New York (1993).
Bih et al. (1999). J. Biol. Chem. 274, 22884-22894.
Choi et al., GenBank accession number AF200816 (1999).
Christensen et al. (1992) Plant mol. Biol. 18, 675-689.
Christou et al, (1990). Trends Biotechnology 8, 145.
Cordera et al. (1994) The Plant Journal 6,141.
Cornejo et al. (1993) Plant Mol. Biol. 23, 567-581.
Cornelissen et al. (1986) EMBO J. 5, 37-40.
Crickmore et al. (1998) Microbiol. Mol. Biol Rev. 62(3), 807-13.
Datta et al., Bio/Technology 8, 736-740 (1990).
Deblaere et al. (1987) Methods In Enzymology 153, 277-292
De Pater et al., 1992, Plant J. 2, 834-844.
Depicker et al., 1982, J. Molec. Appl. Genetics 1, 561-573.
Dulmage, H.T., "Production of Bacteria for Biological Control of Insects" in Biological Control in Crop Production, Ed. Paparizas, D.C., Osmun Publishers, Totowa, N.J., USA, pp.129-141 (1981).
English et al., insect Biochem. Molec. Biol. 24, 1025-1035 (1994).
Estruch et al., (1996), Proc Natl Acad Sci USA 93, 5389-94.
Franck et al., Cell 21, 285-294 (1980)
French etal., Anal.Biochem. 156, 417-423 (1986).
Ffrench-Constant and Bowen (2000) Cell Mol Life Sci 57, 828-33.
Fromm et al., Bio/Technology 8, 833-839 (1990).
Gardner et al., Nucleic Acids Research 9, 2871-2887 (1981)
Ge et al., J. Biol. Chem. 266, 17954-17958 (1991)
Gielen et al., EMBO J 3, 835-845 (1984).
Gordon-Kamm et al., The Plant Cell 2, 603-618 (1990).
Gould et al., Plant Physiol. 95, 426-434 (1991).
Harpster et al., (1988), Molecular and General Genetics 212, 182-190.
Hesse et al. (1989), EMBO J. 8 2453-2461.
Hinchee et al. (1988) Biol/Technology 6, 915.
Ho et al. (1989). Gene 77, 51-59.
Höfte et al., Appl. and Environm. Microbiol. 54, 2010-2017 (1988)
Höfte and Whiteley, Microbiological Review 53, 242-265 (1989).
Hull and Howell, Virology 86, 482-493 (1987)
Itakura et al.(1977). Science 198, 1056-1063.
Jansens et al. (1997) Crop Science 37,1616-1624.
Keil et al. (1986), Nud. Acids Res.14, 5641-5650.
Klösgen et al. (1989), Mol. Gen. Genet. 217, 155-161.
Klösgen and Weil (1991). Mol. Gen. Genet. 225, 297-304.
Kota et al. (1999) Proc. Natl. Acad. Sci. USA 96, 1840-1845.
Krebbers et al. (1988) Plant Molec. Biol. 11, 745-759.
Last et al. (1990) Theor. Appl. Genet. 81, 581-588.
Leredus et al., Bio/Technology 10, 418 (1992).
Mahillon etal, FEMS Microbiol. Letters 60, 205-210 (1989).
Maxam and Gilbert, Methods in Enzymol. 65, 499-560 (1980).
McBride et al., 1995, Bio/Technology 13; 362
Morris et al. (1999), Biochem. Biophys. Res. Commun. 255, 328-333.
Murray et al., M., Nucleic Acids Research 17(2), 477-498 (1989).
Neuhaus & Rogers (1998), Plant Mol. Biol. 38, 127-144.
Odell et al. (1985) Nature 313, 810-812.
Oelmuller et al., Mol. Gen. Genet. 237, 281-272 (1993).
Park et al. (1997), J. Biol. Chem. 272, 6876-6881.
Sanfacon et al. (1991), Genes and Development 5,141-149.
Sanger et al., Proc. Natl. Acad. Sci, USA. 74(12), 5463-5467 (1977).
Schnepf et al.(1985). Journal of Biological Chemistry 260, 6264.
Schnepf et al. (1998). Microbiol. Mol. Biol. Rev. 62(3). 775-806.
Shcherban et al. (1995), Proc. Natl. Acad. Sci USA 92, 9245-9249.
Shimamoto et al., Nature 338, 274-276 (1989).
Stanssens et al., Nucleic Acids Research 12, 4441-4454 (1989).
Sutliff et al. (1991) Plant Molec. Biol. 16. 579-591.
Tavladoraki et al. (1998), FEBS Lett. 426, 62-66.
Terashima et al. (1999), Appl. Microbiol. Biotechnol. 52, 516-523.
Thompson et al. (1987), EMBO J. 6, 2519-2523.
Vaeck et al., 1987, Nature 328, 33-37.
Van Den Broeck et al., 1985, Nature 313, 358.
Vanderzant, J. Econ. Entornol. 55, p.140 (1962).
Van Rie et al., Science 247, 72 (1990).
Velten et al., J., EMBO J 3, 2723-2730 (1984).
Velten and Schell, Nucleic Acids Research 13, 6981-6998 (1985)
Verdaguer et al., Plant Mol. Biol. 31, 1129-1139 (1996).
Verdaguer et al., Plant Mol. Biol. 37, 1055-1067 (1998).
Visser et al., "Domain-Structure Studies of Bacillus thuringiensis Crystal Proteins: A Genetic Approach". In Bacillus thuringiensis, An Environmental Biopesticide: Theory and Practice, pp.71-88, eds. Entwistle, P.F., Cory, J.S., Bailey, M.J. and Higgs, S., John Wiley and Sons. New York (1993).
Wada et al. (1990). Nucl. Acids Res. 18, 2367-1411.
Waterfield et al. (2001) Trends Microbiol 9, 185-91.
White et al.(1989). Trends in Genet 5, 185-189.
Wong et al.(1992), Plant Molec. Biol.20.81-93.
Zhang et al. (1991) The Plant Cell 3, 1155-1165.

## Claims (Claims for the following Contracting State(s): AT, BE, CH, DE, DK, ES, FR, GB, GR, IT, LI, LU, MC, NL SE)

1. A nucleic acid coding sequence encoding an insecticidal Cry2Ae protein consisting of the amino acid sequence of the protein of SEQ ID No. 2 from amino acid position 1 to an amino acid position between amino acid positions 624 and 632 of SEQ ID No. 2.

2. A nucleic acid coding sequence encoding an insecticidal Cry2Ae protein consisting of the amino acid sequence of the protein of SEQ ID No. 2 from an amino acid position between amino acid positions 1 and 51 to an amino acid position between amino acid positions 624 and 632 in SEQ ID No. 2.

3. A nucleic acid coding sequence encoding an insecticidal Cry2Ae protein consisting of the amino acid sequence of the protein of SEQ ID No. 2 from an amino acid position between amino acid positions 1 and 51 to amino acid position 632 in SEQ ID No. 2.

4. The nucleic acid sequence of any one of claims 1 to 3, wherein said nucleic acid sequence is DNA.

5. The nucleic acid sequence of claim 4, comprising an artificial DNA sequence having a different codon usage compared to the naturally occurring DNA sequence but encoding the same protein.

6. A DNA coding sequence encoding an insecticidal protein comprising the amino acid sequence of the protein of SEQ ID No. 2 from an amino acid position between amino acid position 1 and amino acid position 51 to an amino acid position between amino acid position 624 and amino acid position 632, wherein said coding sequence comprises the nucleotide sequence of SEQ ID NO:3 or SEQ ID NO: 5 from nucleotide position 153 to nucleotide position 1880.

7. A DNA coding sequence encoding an insecticidal protein comprising the amino acid sequence of the protein of SEQ ID No. 2 from an amino acid position between amino acid position 1 and amino acid position 51 to amino acid position 632, wherein said coding sequence comprises the nucleotide sequence of SEQ ID NO:3 or SEQ ID NO: 5 from nucleotide position 153 to nucleotide position. 1880.

8. A DNA coding sequence encoding an insecticidal protein comprising the amino acid sequence of the protein of SEQ ID No. 2 from amino acid position 1 to an amino acid position between amino acid positions 624 and 632, wherein said coding sequence comprises the nucleotide sequence of SEQ ID NO:3 or SEQ ID NO: 5 from nucleotide position 153 to nucleotide position 1880.

9. A DNA which comprises the nucleotide sequence of SEQ ID No. 3 or 5.

10. The nucleic acid sequence of any one of claims 1 to 8 or a nucleic acid sequence encoding the protein of SEQ ID NO:2 or an insecticidal portion thereof, wherein the encoded protein starts with a Met-Asp or Met-Ala dipeptide for optimal translation initiation, by the insertion of a codon encoding an Asp or Ala amino acid downstream of the start codon as a new second codon.

11. The nucleic acid sequence of any one of claims 1 to 10, which also contains a DNA encoding a targeting or transit peptide for targeting to the vacuole, chloroplast, mitochondrium, plastid, or for secretion.

12. The nucleic acid sequence of claim 11 which further comprises a DNA encoding a chloroplast transit peptide.

13. An insecticidal protein, consisting of the amino acid sequence of the protein of SEQ ID No. 2 from amino acid position 1 to an amino acid position between amino acid positions 624 and 632 of SEQ ID No. 2.

14. An insecticidal protein consisting of the amino acid sequence of SEQ ID No. 2 from an amino acid position between amino acid positions 1 and 51 to an amino acid position between amino acid positions 624 and 632 in SEQ ID No. 2.

15. An insecticidal protein consisting of the amino acid sequence of the protein of SEQ ID No. 2 from an amino acid position between amino acid positions 1 and 51 to amino acid position 632 in SEQ ID No. 2.

16. An insecticidal protein comprising the amino acid sequence of SEQ ID No. 4.

17. The protein of any one of claims 13 to 16, or an insecticidal protein comprising the amino acid sequence of SEQ ID No.2, or an insecticidal protein comprising the amino acid sequence of SEQ 10 No.2 from amino acid position 50 to amino acid position 625, which is modified to start with a Met-Asp or Met-Ala dipeptide, by addition of an Asp or Ala amino acid downstream of the Met start amino acid.

18. The protein of claim 17, which further comprises a chloroplast transit peptide.

19. A chimeric gene consisting of a coding sequence and a plant-expressible promoter, wherein said coding sequence is the nucleic acid sequence of any one of claims 1 to 12, and wherein said coding sequence is under the control of said plant-expressible promoter.

20. A chimeric gene comprising the following operably-linked elements:
a) a 35S promoter derived from Cauliflower Mosaic Virus;
b) a leader sequence from the chlorophyl a/b binding protein gene from Petunia;
c) the DNA coding sequence of SEQ ID NO:3; and
d) a 3' transcript termination and polyadenylation region of the 35S gene from Cauliflower Mosaic Virus.

21. The chimeric gene of claim 20, wherein said chimeric gene also comprises a DNA encoding the TpssuAt transit peptide allowing chloroplast targeting inserted at the 5' end of the *cry2Ae* coding sequence.

22. Plant cells, plants or seeds transformed to comprise the chimeric gene of any one of claim 19 to 21.

23. A plant, plant cell or seed comprising as first chimeric gene:
a) the chimeric gene of any one of claims 19 to 21, or
b) a chimeric gene comprising a DNA encoding the protein of SEQ ID NO:2 under the control of a plant-expressible promoter, or
c) a chimeric gene comprising a DNA encoding an insecticidal protein comprising the amino acid sequence of SEQ ID NO:2 from amino acid position 50 to amino acid position 625, under the control of a plant-expressible promoter; and
as second chimeric gene a chimeric gene comprising a DNA encoding an insecticidal protein selected from: a Cry1F protein or a toxic fragment thereof; a hybrid protein derived from a Cry1F protein, a Cry1A-Cry1 F hybrid protein; a Cry1A-type protein or a toxic fragment thereof, a Cry1Ac protein or a Cry1Ab-Cry1Ac hybrid protein; a VIP3Aa protein or a toxic fragment thereof, or insecticidal proteins from Xhenorhabdus, Serratia or Photorhabdus species strains.

24. A transgenic cotton plant comprising an insecticidal protein comprising the amino acid sequence of SEQ ID NO:2, or comprising the amino acid sequence of SEQ ID NO:2 from amino acid position 50 to amino acid position 625, as first insect control protein and the Cry1Ac or VIP3Aa protein as second insect control protein.

25. The plant cells, plants or seeds of claim 22 or 23 which are selected from the group consisting of: corn, cotton, rice, tobacco, oilseed rape, Brassica species, eggplant, soybean, potato, sunflower, tomato, sugarcane, tea, beans, strawberry, clover, cucumber, watermelon, pepper, oat, barley, wheat, dahlia, gladiolus, chrysanthemum, sugarbeet, sorghum, alfalfa, and peanut.

26. A micro-organism, transformed to comprise the nucleic acid sequence of any one of claims 1 to 12.

27. A process for rendering a plant resistant to an insect, comprising transforming plant cells with the chimeric gene of any one of claim 19 to 21, and regenerating transformed plants from such cells which are resistant to insects.

28. The process of claim 27, wherein said insect is selected from the group consisting of: Heliothis virescens, Helicoverpa zea, Helicoverpa armigera, Anticarsia gemmatalis and Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis, and Scirpophaga innotata.

29. Use of the protein of any one of claims 13 to 18 to control insects selected from the group consisting of: Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis, and Scirpophaga innotata.

30. Use of the protein of any one of claims 13 to 18, or an insecticidal protein comprising the sequence of SEQ ID NO:2, or comprising the sequence of SEQ ID NO:2 from amino acid position 50 to amino acid position 625, to control an insect selected from the group consisting of: Helicoverpa armigera, Anticarsia gemmatalis, or Sesamia nonagrioides.

31. Use of the protein of any one of claims 13 to 18, or an insecticidal protein comprising the sequence of SEQ ID NO:2, or comprising the sequence of SEQ ID NO:2 from amino acid position 50 to amino acid position 625, to control an insect selected from the group consisting of: Chilo suppressalis, Chilo partellus, Scirpophaga incertulas, Sesamia inferens, Cnaphalocrocis medinalis, Marasmia patnalis, Marasmia exigua, Marasmia ruralis, or Scirpophaga innotata.

32. A process for rendering plants resistant to Helicoverpa armigera, Anticarsia gemmatalis, or Sesamia nonagrioides, comprising transforming plant cells with the chimeric gene of any one of claims 19 to 21, or a chimeric gene comprising: a) a DNA encoding an insecticidal protein comprising the amino acid sequence of SEQ ID NO: 2 under the control of a plant-expressible promoter, or b) a DNA encoding an insecticidal protein comprising the sequence of SEQ ID NO:2 from amino acid position 50 to amino acid position 625 under the control of a plant-expressible promoter, and regenerating transformed plants from such cells.

33. A process for rendering plants resistant to Chilo suppressalis, Chilo partellus, Scirpophaga incertulas, Sesamia inferens, Cnaphalocrocis medinalis, Marasmia patnalis, Marasmia exigua, Marasmia ruralis, or Scirpophaga innotata, comprising transforming plant cells with the chimeric gene of any one of claims 19 to 21, or a chimeric gene comprising: a) a DNA sequence encoding an insecticidal protein comprising the amino acid sequence of SEQ ID NO: 2 under the control of a plant-expressible promoter, or b) a DNA encoding an insecticidal protein comprising the sequence of SEQ ID NO:2 from amino acid position 50 to amino acid position 625 under the control of a plant-expressible promoter, and regenerating transformed plants from such cells.

34. A method for controlling insects comprising expressing in transformed plant cells an insecticidally effective amount of the protein of any one of claims 13 to 18, to control: Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis, or Scirpophaga innotata.

35. Use of the protein of any one of claims 13 to 18 or an insecticidal protein comprising the amino acid sequence of SEQ ID NO:2 or 4, or comprising the amino acid sequence of SEQ ID NO:2 from amino acid position 50 to amino acid position 625, with another insect control protein, which does not recognize at least one binding site recognized by a Cry2Ae protein.

36. A transgenic plant comprising a DNA encoding the protein of any one of claims 13 to 18, or an insecticidal protein comprising the amino acid sequence of SEQ ID NO:2 or 3, or comprising the amino acid sequence of SEQ ID NO:2 from amino acid position 50 to amino acid position 625, which has also been transformed with a DNA encoding a protein conferring resistance to a herbicide based on glufosinate or glyphosate.

## Claims (Claims for the following Contracting State(s): TR)

1. A nucleic acid sequence, encoding an insecticidal Cry2Ae protein comprising the amino acid sequence of the protein of SEQ ID No. 2 or an insecticidal portion thereof, or an insecticidal protein having at least 99 % sequence identity thereto, wherein said insecticidal portion is that smallest portion of SEQ ID No. 2 retaining insecticidal activity that can be obtained by trypsin or chymotrypsin digestion.

2. The nucleic acid sequence of claim 1, encoding an insecticidal Cry2Ae protein comprising the amino acid sequence of the protein of SEQ ID No. 2 from an amino acid position between amino acid positions 1 and 51 to an amino acid position between amino acid positions 624 and 632 in SEQ ID No. 2.

3. The nucleic acid sequence of claim 1, encoding an insecticidal Cry2Ae protein comprising the amino acid sequence from an amino acid position between amino acid positions 1 and 51 to amino acid position 632 in SEQ ID No. 2.

4. The nucleic acid sequence of claim 1, encoding an insecticidal Cry2Ae protein comprising the amino acid sequence from amino acid position 1 to an amino acid position between amino acid positions 624 and 632 in SEQ ID No. 2.

5. The nucleic acid sequence of any one of claims 1 to 4, wherein said nucleic acid sequence is DNA.

6. The nucleic acid sequence of claim 5, comprising an artificial DNA sequence having a different codon usage compared to the naturally occurring DNA sequence but encoding the same protein or its insecticidal portion.

7. The nucleic acid sequence of claim 6 which comprises the nucleotide sequence of SEQ ID No. 3.

8. The nucleic acid sequence of claim 6 which comprises the nucleotide sequence of SED ID No. 5.

9. The nucleic acid of any one of claims 1 to 8, wherein the encoded protein starts with a Met-Asp or Met-Ala dipeptide for optimal translation initiation, by the insertion of a codon encoding an Asp or Ala amino acid downstream of the start codon as a new second codon.

10. A protein comprising the amino acid sequence of the protein of SEQ ID No. 2 or an insecticidal portion thereof, or a protein having a sequence identity of at least 99 % thereto, wherein said insecticidal portion is that smallest portion of SEQ ID No. 2 retaining insecticidal activity that can be obtained by trypsin or chymotrypsin digestion.

11. The protein of claim 10, comprising the amino acid sequence of SEQ ID No. 2 from an amino acid position between amino acid positions 1 and 51 to an amino acid position between amino acid positions 624 and 632 in SEQ ID No. 2.

12. The protein of claim 10, comprising the amino acid sequence of SEQ ID No. 2 from an amino acid position between amino acid positions 1 and 51 to amino acid positions 632 in SEQ ID No. 2.

13. The protein of claim 10, comprising the amino acid sequence of SEQ ID No. 2 from amino acid position 1 to an amino acid position between amino acid positions 624 and 632 in SEQ ID No. 2.

14. The protein of any one of claims 10 to13, comprising the amino acid sequence of SEQ ID No. 4.

15. The protein of any one of claims 10 to 13, wherein said protein starts with a Met-Asp or Met-Ala dipeptide for optimal translation initiation, by the insertion of a codon encoding an Asp or Ala amino acid downstream of the start codon as a new second codon.

16. The protein of any one of claims 10 to 15, which includes a transit peptide for chloroplast targeting.

17. A chimeric gene comprising the nucleic acid sequence of any one of claims 1 to 9, under the control of a plant-expressible promoter.

18. The chimeric gene of claim 17 which further comprises a DNA encoding a targeting or transit peptide for targeting to the vacuole, mitochondrium, chloroplast, plastid, or for secretion.

19. The chimeric gene of claim 18, wherein said further DNA encodes a chloroplast transit peptide.

20. A chimeric gene comprising the following operably-linked elements:
a) a 35S promoter derived from Cauliflower Mosaic Virus;
b) a leader sequence from the chlorophyl a/b binding protein gene from Petunia;
c) the DNA coding sequence of SEQ ID NO:3; and
d) a 3' transcript termination and polyadenylation region of the 35S gene from Cauliflower Mosaic Virus.

21. The chimeric gene of claim 20, wherein said chimeric gene also comprises a DNA encoding the TpssuAt transit peptide allowing chloroplast targeting inserted at the 5' end of the *cry2Ae* coding sequence.

22. Plant cells, plants or seeds transformed to comprise the chimeric gene of any one of claims 17 to 21.

23. The plant cells, plants or seeds of claim 22 which are selected from the group consisting of: corn, cotton, rice, tobacco, oilseed rape, Brassica species, eggplant, soybean, potato, sunflower, tomato, sugarcane, tea, beans, strawberry, clover, cucumber, watermelon, pepper, oat, barley, wheat, dahlia, gladiolus, chrysanthemum, sugarbeet, sorghum, alfalfa, and peanut.

24. A micro-organism, transformed to comprise the nucleic acid sequence of any one of claims 1 to 9.

25. A process for rendering a plant resistant to an insect, comprising transforming plant cells with the chimeric gene of any one of claims 17 to 21, and regenerating transformed plants from such cells which are resistant to insects.

26. The process of claim 25, wherein said insect is selected from the group consisting of: Heliothis virescens, Helicoverpa zea, Helicoverpa armigera, Anticarsia gemmatalis and Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis, and Scirpophaga innotata.

27. Use of the protein of any one of claims 10 to 16 to control insects selected from the group consisting of: Heliothis virescens, Helicoverpa zea, Helicoverpa armigera, Anticarsia gemmatalis and Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis, and Scirpophaga innotata.

28. A plant, plant cell or seed comprising the chimeric gene of any one of claims 17 to 19 and a second chimeric gene comprising a DNA encoding an insecticidal protein selected from: a Cry1F protein or a toxic fragment thereof; a hybrid protein derived from a Cry1 F protein, a Cry1 A-Cry1 F hybrid protein; a Cry1 A-type protein or a toxic fragment thereof, a Cry1Ac protein or a Cry1Ab-Cry1Ac hybrid protein; a VIP3Aa protein or a toxic fragment thereof, or insecticidal proteins from Xhenorhabdus, Serratia or Photorhabdus species strains.

29. A transgenic cotton plant comprising the protein of any one of claims 10 to 13, 15 or 16 as first insect control protein and the Cry1Ac or VIP3Aa protein as second insect control protein.

30. Use of the protein of any one of claims 10 to 16 with another insect control protein, which does not recognize at least one binding site recognized by such Cry2Ae protein.

31. A transgenic plant comprising a DNA encoding the protein of any one of claims 10 to 13, 15 or 16, which has also been transformed with a DNA encoding a protein conferring resistance to a herbicide based on glufosinate or glyphosate.

## Patentansprüche (Patentansprüche für folgende(n) Vertragsstaat(en): AT, BE, CH, DE, DK, ES, FR, GB, GR, IT, LI, LU, MC, NL SE)

1. Nukleinsäure-Codiersequenz, die für ein insektizides Cry2Ae-Protein codiert und die aus der Aminosäuresequenz des Proteins gemäß SEQ ID No: 2 von der Aminosäureposition 1 bis zu einer Aminosäureposition zwischen den Aminosäurepositionen 624 und 632 gemäß SEQ ID NO: 2 besteht.

2. Nukleinsäure-Codiersequenz, die für ein insektizides Cry2Ae-Protein codiert und die aus der Aminosäuresequenz des Proteins gemäß SEQ ID No: 2 von einer Aminosäureposition zwischen den Aminosäurepositionen 1 und 51 bis zu einer Aminosäureposition zwischen den Aminosäurepositionen 624 und 632 in SEQ ID NO: 2 besteht.

3. Nukleinsäure-Codiersequenz, die für ein insektizides Cry2Ae-Protein codiert und die aus der Aminosäuresequenz des Proteins gemäß SEQ ID No: 2 von einer Aminosäureposition zwischen den Aminosäurepositionen 1 und 51 bis zur Aminosäureposition 632 in SEQ ID NO: 2 besteht.

4. Nukleinsäuresequenz nach einem der Ansprüche 1 bis 3, wobei es sich bei der Nukleinsäuresequenz um DNA handelt.

5. Nukleinsäuresequenz nach Anspruch 4, umfassend eine künstliche DNA-Sequenz mit einem unterschiedlichen "Codon Usage" im Vergleich zu der natürlich vorkommenden DNA-Sequenz, die jedoch für dasselbe Protein codiert.

6. DNA-Codiersequenz, die für ein insektizides Protein codiert und die die Aminosäuresequenz des Proteins gemäß SEQ ID NO: 2 von einer Aminosäureposition zwischen der Aminosäureposition 1 und der Aminosäureposition 51 bis zu einer Aminosäureposition zwischen der Aminosäureposition 624 und der Aminosäureposition 632 umfaßt, wobei diese Codiersequenz die Nukleotidsequenz gemäß SEQ ID NO: 3 oder SEQ ID NO: 5 von der Nukleotidposition 153 bis zur Nukleotidposition 1880 umfaßt.

7. DNA-Codiersequenz, die für ein insektizides Protein codiert und die die Aminosäuresequenz des Proteins gemäß SEQ ID NO: 2 von einer Aminosäureposition zwischen der Aminosäureposition 1 und der Aminosäureposition 51 bis zur Aminosäureposition 632 umfaßt, wobei diese Codiersequenz die Nukleotidsequenz gemäß SEQ ID NO: 3 oder SEQ ID NO: 5 von der Nukleotidposition 153 bis zur Nukleotidposition 1880 umfaßt.

8. DNA-Codiersequenz, die für ein insektizides Protein codiert und die die Aminosäuresequenz des Proteins gemäß SEQ ID NO: 2 von der Aminosäureposition 1 bis zu einer Aminosäureposition zwischen den Aminosäurepositionen 624 und 632 umfaßt, wobei diese Codiersequenz die Nukleotidsequenz gemäß SEQ ID NO: 3 oder SEQ ID NO: 5 von der Nukleotidposition 153 bis zur Nukleotidposition 1880 umfaßt.

9. DNA, die die Nukleotidsequenz gemäß SEQ ID NO: 3 oder 5 umfaßt.

10. Nukleinsäuresequenz nach einem der Ansprüche 1 bis 8 oder Nukleinsäuresequenz, die für das Protein gemäß SEQ ID NO: 2 oder einen insektiziden Abschnitt davon codiert, wobei das codierte Protein mit einem Met-Asp oder Met-Ala-Dipeptid für einen optimalen Translationsstart beginnt, und zwar durch Insertion eines Codons, das für eine Aminosäure Asp oder Ala codiert, stromabwärts des Start-Codons als neues zweites Codon.

11. Nukleinsäuresequenz nach einem der Ansprüche 1 bis 10, die auch eine DNA enthält, die für ein Zielsteuerungs- oder Transitpeptid für die Zielsteuerung an die Vakuole, an den Chloroplasten, an das Mitochondrium, an den Plastiden oder für die Sekretion codiert.

12. Nukleinsäuresequenz nach Anspruch 11, die weiterhin eine DNA umfaßt, die für ein Chloroplastentransitpeptid codiert.

13. Insektizides Protein, das aus der Aminosäuresequenz des Proteins gemäß SEQ ID NO: 2 von der Aminosäureposition 1 bis zu einer Aminosäureposition zwischen den Aminosäurepositionen 624 und 632 gemäß SEQ ID NO: 2 besteht.

14. Insektizides Protein, das aus der Aminosäuresequenz gemäß SEQ ID NO: 2 von einer Aminosäureposition zwischen den Aminosäurepositionen 1 und 51 bis zu einer Aminosäureposition zwischen den Aminosäurepositionen 624 und 632 in SEQ ID NO: 2 besteht.

15. Insektizides Protein, das aus der Aminosäuresequenz des Proteins gemäß SEQ ID NO: 2 von einer Aminosäureposition zwischen den Aminosäurepositionen 1 und 51 bis zur Aminosäureposition 632 in SEQ ID NO: 2 besteht.

16. Insektizides Protein, das die Aminosäuresequenz gemäß SEQ ID NO: 4 umfaßt.

17. Protein nach einem der Ansprüche 13 bis 16 oder insektizides Protein, das die Aminosäuresequenz gemäß SEQ ID NO: 2 umfaßt, oder insektizides Protein, das die Aminosäuresequenz gemäß SEQ ID NO: 2 von der Aminosäureposition 50 bis zur Aminosäureposition 625 umfaßt, das dahingehend modifiziert ist, daß es mit einem Met-Asp- oder Met-Ala-Dipeptid beginnt, und zwar durch Hinzufügen einer Aminosäure Asp oder Ala stromabwärts der Start-Aminosäure Met.

18. Protein nach Anspruch 17, das weiterhin ein Chloroplastentransitpeptid umfaßt.

19. Chimäres Gen, das aus einer Codiersequenz und einem in Pflanzen exprimierbaren Promoter besteht, wobei es sich bei der Codiersequenz um die Nukleinsäuresequenz nach einem der Ansprüche 1 bis 12 handelt und wobei die Codiersequenz unter der Kontrolle des in Pflanzen exprimierbaren Promoters steht.

20. Chimäres Gen, das die folgenden operativ verknüpften Elemente umfaßt:
a) einen vom Blumenkohlmosaikvirus abgeleiteten 35S-Promoter;
b) eine Leitsequenz von dem "chlorophyl a/b binding protein"-Gen aus der Petunie;
c) die DNA-Codiersequenz der SEQ ID NO: 3; und
d) eine 3'-Transkriptterminations- und -polyadenylierungsregion vom 35S-Gen des Blumenkohlmosaikvirus.

21. Chimäres Gen nach Anspruch 20, wobei das chimäre Gen zusätzlich eine DNA, die für das TpssuAt-Transitpeptid, das eine Zielsteuerung an den Chloroplasten ermöglicht, codiert, am 5'-Ende der cry2Ae-Codiersequenz insertiert enthält.

22. Pflanzenzellen, Pflanzen oder Samen, die dahingehend transformiert sind, daß sie das chimäre Gen nach einem der Ansprüche 19 bis 21 umfassen.

23. Pflanze, Pflanzenzelle oder Samen, umfassend als erstes chimäres Gen:
a) das chimäre Gen nach einem der Ansprüche 19 bis 21, oder
b) ein chimäres Gen, das eine DNA, die für das Protein gemäß SEQ ID NO: 2 codiert, unter der Kontrolle eines in Pflanzen exprimierbaren Promoters umfaßt, oder
c) ein chimäres Gen, das eine DNA, die für ein insektizides Protein, das die Aminosequenz gemäß SEQ ID NO: 2 von der Aminosäureposition 50 bis zur Aminosäureposition 625 umfaßt, codiert, unter der Kontrolle eines in Pflanzen exprimierbaren Promoters umfaßt; und
als zweites chimäres Gen ein chimäres Gen, das eine DNA, die für ein insektizides Protein aus der Reihe: ein Cry1F-Protein oder ein toxisches Fragment davon; ein Hybridprotein, das sich von einem Cry1F-Protein ableitet, ein CrylA-Cry1F-Hybridprotein, ein Protein des CrylA-Typs oder ein toxisches Fragment davon; ein Cry1Ac-Protein oder ein Cry1Ab-Cry1Ac-Hybridprotein; ein VIP3Aa-Protein und ein toxisches Fragment davon, oder insektizide Proteine von Stämmen der Art Xhenorhabdus, Serratia oder Photorhabdus codiert, umfaßt.

24. Transgene Baumwollpflanze, umfassend ein insektizides Protein, das die Aminosäuresequenz gemäß SEQ ID NO: 2 umfaßt, oder das die Aminosäuresequenz gemäß SEQ ID NO: 2 von der Aminosäureposition 50 bis zur Aminosäureposition 625 umfaßt, als erstes Insektenbekämpfungsprotein sowie das Cry1Ac- oder VIP3Aa-Protein als zweites Insektenbekämpfungsprotein.

25. Pflanzenzellen, Pflanzen oder Samen nach Anspruch 22 oder 23, die aus der folgenden Gruppe stammen: Mais, Baumwolle, Reis, Tabak, Raps, Brassica-Arten, Aubergine, Sojabohne, Kartoffel, Sonnenblume, Tomate, Zuckerrohr, Tee, Bohnen, Erdbeere, Klee, Gurke, Wassermelone, Pfeffer, Hafer, Gerste, Weizen, Dahlie, Gladiole, Chrysantheme, Zuckerrübe, Sorghumhirse, Luzerne und Erdnuß.

26. Mikroorganismus, der dahingehend transformiert ist, daß er die Nukleinsäuresequenz nach einem der Ansprüche 1 bis 12 enthält.

27. Verfahren zum Resistentmachen einer Pflanze gegen ein Insekt, umfassend die Transformation von Pflanzenzellen mit dem chimären Gen nach einem der Ansprüche 19 bis 21 und das Regenerieren der transformierten Pflanzen aus solchen Zellen, die gegen Insekten resistent sind.

28. Verfahren nach Anspruch 27, wobei das Insekt aus der folgenden Gruppe stammt: Heliothis virescens, Helicoverpa zea, Helicoverpa armigera, Anticarsia gemmatalis und Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis und Scirpophaga innotata.

29. Verwendung des Proteins nach einem der Ansprüche 13 bis 18 zur Bekämpfung von Insekten aus der folgenden Gruppe: Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis und Scirpophaga innotata.

30. Verwendung des Proteins nach einem der Ansprüche 13 bis 18 oder eines insektiziden Proteins umfassend die Sequenz gemäß SEQ ID NO: 2 oder umfassend die Sequenz gemäß SEQ ID NO: 2 von der Aminosäureposition 50 bis zur Aminosäureposition 625 zur Bekämpfung eines Insekts aus der folgenden Gruppe: Helicoverpa armigera, Anticarsia gemmatalis oder Sesamia nonagrioides.

31. Verwendung des Proteins nach einem der Ansprüche 13 bis 18 oder eines insektiziden Proteins umfassend die Sequenz gemäß SEQ ID NO: 2 oder umfassend die Sequenz gemäß SEQ ID NO: 2 von der Aminosäureposition 50 bis zur Aminosäureposition 625 zur Bekämpfung eines Insekts aus der folgenden Gruppe: Chilo suppressalis, Chilo partellus, Scirpophaga incertulas, Sesamia inferens, Cnaphalocrocis medinalis, Marasmia patnalis, Marasmia exigua, Marasmia ruralis oder Scirpophaga innotata.

32. Verfahren zum Resistentmachen von Pflanzen gegen Helicoverpa armigera, Anticarsia gemmatalis oder Sesamia nonagrioides, umfassend das Transformieren von Pflanzenzellen mit dem chimären Gen nach einem der Ansprüche 19 bis 21 oder mit einem chimären Gen umfassend: a) eine DNA, die für ein insektizides Protein, das die Aminosäuresequenz gemäß SEQ ID NO: 2 umfaßt, unter der Kontrolle eines in Pflanzen exprimierbaren Promoters codiert, oder b) eine DNA, die für ein insektizides Protein, das die Sequenz gemäß SEQ ID NO: 2 von der Aminosäureposition 50 bis zur Aminosäureposition 625 umfaßt, unter der Kontrolle eines in Pflanzen exprimierbaren Promoters codiert, und das Regenerieren von transformierten Pflanzen aus solchen Zellen.

33. Verfahren zum Resistentmachen von Pflanzen gegen Chilo suppressalis, Chilo partellus, Scirpophaga incertulas, Sesamia inferens, Cnaphalocrocis medinalis, Marasmia patnalis, Marasmia exigua, Marasmia ruralis oder Scirpophaga innotata, umfassend das Transformieren von Pflanzenzellen mit dem chimären Gen nach einem der Ansprüche 19 bis 21 oder mit einem chimären Gen umfassend: a) eine DNA, die für ein insektizides Protein, das die Aminosäuresequenz gemäß SEQ ID NO: 2 umfaßt, unter der Kontrolle eines in Pflanzen exprimierbaren Promoters codiert, oder b) eine DNA, die für ein insektizides Protein, das die Sequenz gemäß SEQ ID NO: 2 von der Aminosäureposition 50 bis zur Aminosäureposition 625 umfaßt, unter der Kontrolle eines in Pflanzen exprimierbaren Promoters codiert, und das Regenerieren von transformierten Pflanzen aus solchen Zellen.

34. Verfahren zur Bekämpfung von Insekten, umfassend das Exprimieren einer insektizidwirksamen Menge des Proteins nach einem der Ansprüche 13 bis 18 in transformierten Pflanzenzellen, um Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis oder Scirpophaga innotata zu bekämpfen.

35. Verwendung des Proteins nach einem der Ansprüche 13 bis 18 oder eines insektiziden Proteins, das die Aminosäuresequenz gemäß SEQ ID NO: 2 oder 4 umfaßt, oder das die Aminosäuresequenz gemäß Sequenz SEQ ID NO: 2 von der Aminosäureposition 50 bis zur Aminosäureposition 625 umfaßt, zusammen mit einem weiteren Insektenbekämpfungsprotein, das mindestens eine von einem Cry2Ae-Protein erkannte Bindungsstelle nicht erkennt.

36. Transgene Pflanze, umfassend eine DNA, die für das Protein nach einem der Ansprüche 13 bis 18 oder für ein insektizides Protein, das die Aminosäuresequenz gemäß SEQ ID NO: 2 oder 3 umfaßt oder das die Aminosäuresequenz gemäß SEQ ID NO: 2 von der Aminosäureposition 50 bis zur Aminosäureposition 625 umfaßt, codiert, die auch mit einer DNA, die für ein Protein, das Resistenz gegenüber einem Herbizid auf Glufosinate- oder Glyphosate-Basis vermittelt, codiert, transformiert worden ist.

## Patentansprüche (Patentansprüche für folgende(n) Vertragsstaat(en): TR)

1. Nukleinsäuresequenz, die für ein insektizides Cry2Ae-Protein, das die Aminosäuresequenz des Proteins gemäß SEQ ID NO: 2 oder einen insektiziden Abschnitt davon umfaßt, oder für ein insektizides Protein mit mindestens 99% Sequenzidentität dazu codiert, wobei der insektizide Abschnitt der kleinste Abschnitt der SEQ ID NO: 2 ist, der noch eine insektizide Aktivität aufweist und der durch Verdauen mit Trypsin oder Chymotrypsin erhältlich ist.

2. Nukleinsäuresequenz nach Anspruch 1, die für ein insektizides Cry2Ae-Protein codiert und die aus der Aminosäuresequenz des Proteins gemäß SEQ ID No: 2 von einer Aminosäureposition zwischen den Aminosäurepositionen 1 und 51 bis zu einer Aminosäureposition zwischen den Aminosäurepositionen 624 und 632 in SEQ ID NO: 2 besteht.

3. Nukleinsäuresequenz nach Anspruch 1, die für ein insektizides Cry2Ae-Protein codiert und die aus der Aminosäuresequenz von einer Aminosäureposition zwischen den Aminosäurepositionen 1 und 51 bis zur Aminosäureposition 632 in SEQ ID NO: 2 besteht.

4. Nukleinsäuresequenz nach Anspruch 1, die für ein insektizides Cry2Ae-Protein, das die Aminosäuresequenz von der Aminosäureposition 1 bis zu einer Aminosäureposition zwischen den Aminosäurepositionen 624 und 632 in SEQ ID NO: 2 umfaßt, codiert.

5. Nukleinsäuresequenz nach einem der Ansprüche 1 bis 4, wobei es sich bei der Nukleinsäuresequenz um DNA handelt.

6. Nukleinsäuresequenz nach Anspruch 5, umfassend eine künstliche DNA-Sequenz mit einem unterschiedlichen "Codon Usage" im Vergleich zu der natürlich vorkommenden DNA-Sequenz, die jedoch für dasselbe Protein oder seinen insektiziden Abschnitt codiert.

7. Nukleinsäuresequenz nach Anspruch 6, die die Nukleotidsequenz gemäß SEQ ID NO: 3 umfaßt.

8. Nukleinsäuresequenz nach Anspruch 6, die die Nukleotidsequenz gemäß SEQ ID NO: 5 umfaßt.

9. Nukleinsäure nach einem der Ansprüche 1 bis 8, wobei das codierte Protein mit einem Met-Asp- oder Met-Ala-Dipeptid für einen optimalen Translationsstart beginnt, und zwar durch Insertion eines Codons, das für eine Aminosäure Asp oder Ala codiert, stromabwärts des Start-Codons als neues zweites Codon.

10. Protein, umfassend die Aminosäuresequenz des Proteins gemäß SEQ ID NO: 2 oder eines insektiziden Abschnitts davon, oder ein Protein mit mindestens 99% Sequenzidentität dazu, wobei der insektizide Abschnitt der kleinste Abschnitt der SEQ ID NO: 2 ist, der noch eine insektizide Aktivität aufweist und der durch Verdauen mit Trypsin oder Chymotrypsin erhältlich ist.

11. Protein nach Anspruch 10, umfassend die Aminosäuresequenz gemäß SEQ ID NO: 2 von einer Aminosäureposition zwischen den Aminosäurepositionen 1 und 51 bis zu einer Aminosäureposition zwischen den Aminosäurepositionen 624 und 632 in SEQ ID NO: 2.

12. Protein nach Anspruch 10, das die Aminosäuresequenz gemäß SEQ ID NO: 2 von einer Aminosäureposition zwischen den Aminosäurepositionen 1 und 51 bis zur Aminosäureposition 632 in SEQ ID NO: 2 umfaßt.

13. Protein nach Anspruch 10, das die Aminosäuresequenz gemäß SEQ ID NO: 2 von der Aminosäureposition 1 bis zu einer Aminosäureposition zwischen den Aminosäurepositionen 624 und 632 in SEQ ID NO: 2 umfaßt.

14. Protein nach einem der Ansprüche 10 bis 13, das die Aminosäuresequenz gemäß SEQ ID NO: 4 umfaßt.

15. Protein nach einem der Ansprüche 10 bis 13, wobei das Protein mit einem Met-Asp- oder Met-Ala-Dipeptid für einen optimalen Translationsstart beginnt, und zwar durch Insertion eines Codons, das für eine Aminosäure Asp oder Ala codiert, stromabwärts des Start-Codons als neues zweites Codon.

16. Protein nach einem der Ansprüche 10 bis 15, das ein Transitpeptid für die Adressierung in den Chloroplasten beinhaltet.

17. Chimäres Gen, das die Nukleinsäuresequenz nach einem der Ansprüche 1 bis 9 unter der Kontrolle eines in Pflanzen exprimierbaren Promoters umfaßt.

18. Chimäres Gen nach Anspruch 17, das weiterhin eine DNA enthält, die für ein Zielsteuerungs- oder Transitpeptid für die Zielsteuerung an die Vakuole, an das Mitochondrium, an den Chloroplasten, an den Plastiden oder für die Sekretion codiert, umfaßt.

19. Chimäres Gen nach Anspruch 18, wobei die weitere DNA für ein Chloroplastentransitpeptid codiert.

20. Chimäres Gen, das die folgenden operativ verknüpften Elemente umfaßt:
a) einen von Blumenkohlmosaikvirus abgeleiteten 35S-Promoter;
b) eine Leitsequenz von dem "chlorophyl a/b binding protein"-Gen aus der Petunie;
c) die DNA-Codiersequenz der SEQ ID NO: 3; und
d) eine 3'-Transkriptterminations- und -polyadenylierungsregion vom 35S-Gen des Blumenkohlmosaikvirus.

21. Chimäres Gen nach Anspruch 20, wobei das chimäre Gen zusätzlich eine DNA, die für das TpssuAt-Transitpeptid, das eine Zielsteuerung an den Chloroplasten ermöglicht, codiert, am 5'-Ende der cry2Ae-Codiersequenz insertiert enthält.

22. Pflanzenzellen, Pflanzen oder Samen, die dahingehend transformiert sind, daß sie das chimäre Gen nach einem der Ansprüche 17 bis 21 umfassen.

23. Pflanzenzellen, Pflanzen oder Samen nach Anspruch 22, die aus der folgenden Gruppe stammen: Mais, Baumwolle, Reis, Tabak, Raps, Brassica-Arten, Aubergine, Sojabohne, Kartoffel, Sonnenblume, Tomate, Zuckerrohr, Tee, Bohnen, Erdbeere, Klee, Gurke, Wassermelone, Pfeffer, Hafer, Gerste, Weizen, Dahlie, Gladiole, Chrysantheme, Zuckerrübe, Sorghumhirse, Luzerne und Erdnuß.

24. Mikroorganismus, der dahingehend transformiert ist, daß er die Nukleinsäuresequenz nach einem der Ansprüche 1 bis 9 enthält.

25. Verfahren zum Resistentmachen einer Pflanze gegen ein Insekt, umfassend die Transformation von Pflanzenzellen mit dem chimären Gen nach einem der Ansprüche 17 bis 21 und das Regenerieren der transformierten Pflanzen aus solchen Zellen, die gegen Insekten resistent sind.

26. Verfahren nach Anspruch 25, wobei das Insekt aus der folgenden Gruppe stammt: Heliothis virescens, Helicoverpa zea, Helicoverpa armigera, Anticarsia gemmatalis und Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis und Scirpophaga innotata.

27. Verwendung des Proteins nach einem der Ansprüche 10 bis 16 zur Bekämpfung von Insekten aus der folgenden Gruppe: Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis und Scirpophaga innotata.

28. Pflanze, Pflanzenzelle oder Samen, umfassend das chimäre Gen nach einem der Ansprüche 17 bis 19 und ein zweites chimäres Gen umfassend eine DNA, die für ein insektizides Protein aus der Reihe: ein Cry1F-Protein oder ein toxisches Fragment davon; ein Hybridprotein, das sich von einem Cry1F-Protein ableitet, ein Cry1A-Cry1F-Hybridprotein, ein Protein des Cry1A-Typs oder ein toxisches Fragment davon; ein Cry1Ac-Protein oder ein Cry1Ab-Cry1Ac-Hybridprotein; ein VIP3Aa-Protein und ein toxisches Fragment davon, oder insektizide Proteine von Stämmen der Art Xhenorhabdus, Serratia oder Photorhabdus codiert.

29. Transgene Baumwollpflanze, die das Protein nach einem der Ansprüche 10 bis 13, 15 oder 16 als erstes Insektenbekämpfungsprotein und das Cry1Ac- oder VIP3Aa-Protein als zweites Insektenbekämpfungsprotein umfaßt.

30. Verwendung des Proteins nach einem der Ansprüche 10 bis 16 zusammen mit einem weiteren Insektenbekämpfungsprotein, das mindestens eine von solch einem Cry2Ae-Protein erkannte Bindungsstelle nicht erkennt.

31. Transgene Pflanze, die eine DNA, die für das Protein nach einem der Ansprüche 10 bis 13, 15 oder 16 codiert, umfaßt und die auch mit einer DNA, die für ein Protein, das Resistenz gegenüber einem Herbizid auf Glufosinate- oder Glyphosate-Basis vermittelt, codiert, transformiert worden ist.

## Revendications (Revendications pour l'(les) Etat(s) contractant(s) suivant(s): AT, BE, CH, DE, DK, ES, FR, GB, GR, IT, LI, LU, MC, NL SE)

1. Séquence codante d'acide nucléique codant pour une protéine insecticide Cry2Ae constituée de la séquence d'acides aminés de la protéine de SEQ ID NO : 2 de la position d'acide aminé 1 à une position d'acide aminé comprise entre les positions d'acides aminés 624 et 632 de SEQ ID NO : 2.

2. Séquence codante d'acide nucléique codant pour une protéine insecticide Cry2Ae constituée de la séquence d'acides aminés de la protéine de SEQ ID NO : 2 d'une position d'acide aminé comprise entre les positions d'acides aminés 1 et 51 à une position d'acide aminé comprise entre les positions d'acides aminés 624 et 632 de SEQ ID NO : 2.

3. Séquence codante d'acide nucléique codant pour une protéine insecticide Cry2Ae constituée de la séquence d'acides aminés de la protéine de SEQ ID NO : 2 d'une position d'acide aminé comprise entre les positions d'acides aminés 1 et 51 à la position d'acide aminé 632 de SEQ ID NO : 2.

4. Séquence d'acide nucléique selon l'une quelconque des revendications 1 à 3, ladite séquence d'acide nucléique étant de l'ADN.

5. Séquence d'acide nucléique selon la revendication 4, comprenant une séquence d'ADN artificielle présentant un usage des codons différent par rapport à la séquence d'ADN naturellement présente, mais codant pour la même protéine.

6. Séquence codante d'ADN codant pour une protéine insecticide comprenant la séquence d'acides aminés de la protéine de SEQ ID NO : 2 d'une position d'acide aminé comprise entre la position d'acide aminé 1 et la position d'acide aminé 51 à une position d'acide aminé comprise entre la position d'acide aminé 624 et la position d'acide aminé 632, ladite séquence codante comprenant la séquence nucléotidique de SEQ ID NO : 3 ou de SEQ ID NO : 5 de la position nucléotidique 153 à la position nucléotidique 1880.

7. Séquence codante d'ADN codant pour une protéine insecticide comprenant la séquence d'acides aminés de la protéine de SEQ ID NO : 2 d'une position d'acide aminé comprise entre la position d'acide aminé 1 et la position d'acide aminé 51 à la position d'acide aminé 632, ladite séquence codante comprenant la séquence nucléotidique de SEQ ID NO : 3 ou de SEQ ID NO : 5 de la position nucléotidique 153 à la position nucléotidique 1880.

8. Séquence codante d'ADN codant pour une protéine insecticide comprenant la séquence d'acides aminés de la protéine de SEQ ID NO : 2 de la position d'acide aminé 1 à une position d'acide aminé comprise entre les positions d'acides aminés 624 et 632, ladite séquence codante comprenant la séquence nucléotidique de SEQ ID NO : 3 ou de SEQ ID NO : 5 de la position nucléotidique 153 à la position nucléotidique 1880.

9. ADN qui comprend la séquence nucléotidique de SEQ ID NO : 3 ou 5.

10. Séquence d'acide nucléique selon l'une quelconque des revendications 1 à 8 ou séquence d'acide nucléique codant pour la protéine de SEQ ID NO : 2 ou une partie insecticide de celle-ci, la protéine codée commençant par un dipeptide Met-Asp ou Met-Ala pour une initiation de la traduction optimale, par l'insertion, en aval du codon d'initiation, d'un codon codant pour un acide aminé Asp ou Ala en tant que nouveau deuxième codon.

11. Séquence d'acide nucléique selon l'une quelconque des revendications 1 à 10, qui contient également un ADN codant pour un peptide d'adressage ou de transit pour l'adressage à la vacuole, au chloroplaste, à la mitochondrie, au plaste, ou pour la sécrétion.

12. Séquence d'acide nucléique selon la revendication 11, qui comprend en outre un ADN codant pour un peptide de transit vers le chloroplaste.

13. Protéine insecticide constituée de la séquence d'acides aminés de la protéine de SEQ ID NO : 2 de la position d'acide aminé 1 à une position d'acide aminé comprise entre les positions d'acides aminés 624 et 632 de SEQ ID NO : 2.

14. Protéine insecticide constituée de la séquence d'acides aminés de SEQ ID NO : 2 d'une position d'acide aminé comprise entre les positions d'acides aminés 1 et 51 à une position d'acide aminé comprise entre les positions d'acides aminés 624 et 632 de SEQ ID NO : 2.

15. Protéine insecticide constituée de la séquence d'acides aminés de la protéine de SEQ ID NO : 2 d'une position d'acide aminé comprise entre les positions d'acides aminés 1 et 51 à la position d'acide aminé 632 de SEQ ID NO : 2.

16. Protéine insecticide comprenant la séquence d'acide aminé de SEQ ID NO : 4.

17. Protéine selon l'une quelconque des revendications 13 à 16, ou protéine insecticide comprenant la séquence d'acides aminés de SEQ ID NO : 2, ou protéine insecticide comprenant la séquence d'acides aminés de SEQ ID NO : 2 de la position d'acide aminé 50 à la position d'acide aminé 625, qui est modifiée de manière à commencer par un dipeptide Met-Asp ou Met-Ala, par l'addition d'un acide aminé Asp ou Ala en aval de l'acide aminé d'initiation Met.

18. Protéine selon la revendication 17, qui comprend en outre un peptide de transit vers le chloroplaste.

19. Gène chimère constitué d'une séquence codante et d'un promoteur exprimable chez les plantes, ladite séquence codante étant la séquence d'acide nucléique selon l'une quelconque des revendications 1 à 12 et ladite séquence codante étant sous le contrôle dudit promoteur exprimable chez les plantes.

20. Gène chimère comprenant les éléments suivants liés de manière opérationnelle :
a) un promoteur 35S issu du virus de la mosaïque du chou-fleur ;
b) une séquence de tête provenant du gène de la protéine de liaison à la chlorophylle a/b de pétunia ;
c) la séquence codante d'ADN de SEQ ID NO : 3 ; et
d) une région 3' de terminaison de la transcription et de polyadénylation du gène 35S du virus de la mosaïque du chou-fleur.

21. Gène chimère selon la revendication 20, ledit gène chimère comprenant également un ADN codant pour le peptide de transit TpssuAt permettant l'adressage au chloroplaste, inséré à l'extrémité 5' de la séquence codante *cry2Ae.*

22. Cellules végétales, plantes ou graines transformées de manière à comprendre le gène chimère selon l'une quelconque des revendications 19 à 21.

23. Plante, cellule végétale ou graine comprenant à titre de premier gène chimère :
a) le gène chimère selon l'une quelconque des revendications 19 à 21, ou
b) un gène chimère comprenant un ADN codant pour la protéine de SEQ ID NO : 2 sous le contrôle d'un promoteur exprimable chez les plantes, ou
c) un gène chimère comprenant un ADN codant pour une protéine insecticide comprenant la séquence d'acides aminés de SEQ ID NO : 2 de la position d'acide aminé 50 à la position d'acide aminé 625, sous le contrôle d'un promoteur exprimable chez les plantes ; et
à titre de deuxième gène chimère, un gène chimère comprenant un ADN codant pour une protéine insecticide choisie parmi : une protéine Cry1F ou un fragment toxique de celle-ci ; une protéine hybride dérivée d'une protéine Cry1F, une protéine hybride Cry1A-Cry1F ; une protéine de type Cry1A ou un fragment toxique de celle-ci, une protéine Cry1Ac ou une protéine hybride Cry1Ab-Cry1Ac ; une protéine VIP3Aa ou un fragment toxique de celle-ci, ou des protéines insecticides de souches des espèces de Xhenorhabdus, Serratia ou Photorhabdus.

24. Plante de coton transgénique comprenant une protéine insecticide comprenant la séquence d'acides aminés de SEQ ID NO : 2, ou comprenant la séquence d'acides aminés de SEQ ID NO : 2 de la position d'acide aminé 50 à la position d'acide aminé 625, à titre de première protéine de lutte contre les insectes et la protéine Cry1Ac ou VIP3Aa à titre de deuxième protéine de lutte contre les insectes.

25. Cellules végétales, plantes ou graines selon la revendication 22 ou 23, qui sont choisies dans le groupe constitué : du maïs, du coton, du riz, du tabac, du colza, d'espèces de Brassica, de l'aubergine, du soja, de la pomme de terre, du tournesol, de la tomate, de la canne à sucre, du thé, des haricots, de la fraise, du trèfle, du concombre, de la pastèque, du poivron, de l'avoine, de l'orge, du blé, du dahlia, du glaïeul, du chrysanthème, de la betterave sucrière, du sorgho, de la luzerne et de l'arachide.

26. Microorganisme transformé de manière à comprendre la séquence d'acide nucléique selon l'une quelconque des revendications 1 à 12.

27. Procédé permettant de rendre une plante résistante à un insecte, comprenant la transformation de cellules végétales par le gène chimère selon l'une quelconque des revendications 19 à 21 et la régénération de plantes transformées qui sont résistantes aux insectes à partir de telles cellules.

28. Procédé selon la revendication 27, ledit insecte étant choisi dans le groupe constitué : d'Heliothis virescens, d'Helicoverpa zea, d'Helicoverpa armigera, d'Anticarsia gemmatalis, d'Ostrinia nubilalis, de Chilo suppressalis, de Chilo partellus, de Scirpophaga incertulas, de Sesamia inferens, de Cnaphalocrocis medinalis, de Marasmia patnalis, de Marasmia exigua, de Marasmia ruralis et de Scirpophaga innotata.

29. Utilisation de la protéine selon l'une quelconque des revendications 13 à 18, afin de lutter contre des insectes choisis dans le groupe constitué : d'Heliothis virescens, d'Helicoverpa zea, d'Helicoverpa armigera, d'Anticarsia gemmatalis, d'Ostrinia nubilalis, de Chilo suppressalis, de Chilo partellus, de Scirpophaga incertulas, de Sesamia inferens, de Cnaphalocrocis medinalis, de Marasmia patnalis, de Marasmia exigua, de Marasmia ruralis et de Scirpophaga innotata.

30. Utilisation de la protéine selon l'une quelconque des revendications 13 à 18, ou d'une protéine insecticide comprenant la séquence de SEQ ID NO : 2, ou comprenant la séquence de SEQ ID NO : 2 de la position d'acide aminé 50 à la position d'acide aminé 625, afin de lutter contre un insecte choisi dans le groupe constitué : d'Helicoverpa armigera, d'Anticarsia gemmatalis, et de Sesamia nonagrioides.

31. Utilisation de la protéine selon l'une quelconque des revendications 13 à 18, ou d'une protéine insecticide comprenant la séquence de SEQ ID NO : 2, ou comprenant la séquence de SEQ ID NO : 2 de la position d'acide aminé 50 à la position d'acide aminé 625, afin de lutter contre un insecte choisi dans le groupe constitué : de Chilo suppressalis, de Chilo partellus, de Scirpophaga incertulas, de Sesamia inferens, de Cnaphalocrocis medinalis, de Marasmia patnalis, de Marasmia exigua, de Marasmia ruralis et de Scirpophaga innotata.

32. Procédé permettant de rendre des plantes résistantes à Helicoverpa armigera, Anticarsia gemmatalis ou Sesamia nonagrioides, comprenant la transformation de cellules végétales par le gène chimère selon l'une quelconque des revendications 19 à 21, ou par un gène chimère comprenant : a) un ADN codant pour une protéine insecticide comprenant la séquence d'acides aminés de SEQ ID NO : 2 sous le contrôle d'un promoteur exprimable chez les plantes, ou b) un ADN codant pour une protéine insecticide comprenant la séquence de SEQ ID NO : 2 de la position d'acide aminé 50 à la position d'acide aminé 625 sous le contrôle d'un promoteur exprimable chez les plantes, et la régénération de plantes transformées à partir de telles cellules.

33. Procédé permettant de rendre des plantes résistantes à Chilo suppressalis, Chilo partellus, Scirpophaga incertulas, Sesamia inferens, Cnaphalocrocis medinalis, Marasmia patnalis, Marasmia exigu, Marasmia ruralis ou Scirpophaga innotata, comprenant la transformation de cellules végétales par le gène chimère selon l'une quelconque des revendications 19 à 21, ou par un gène chimère comprenant : a) une séquence ADN codant pour une protéine insecticide comprenant la séquence d'acides aminés de SEQ ID NO : 2 sous le contrôle d'un promoteur exprimable chez les plantes, ou b) un ADN codant pour une protéine insecticide comprenant la séquence de SEQ ID NO : 2 de la position d'acide aminé 50 à la position d'acide aminé 625 sous le contrôle d'un promoteur exprimable chez les plantes, et la régénération de plantes transformées à partir de telles cellules.

34. Méthode de lutte contre les insectes, comprenant l'expression d'une quantité efficace du point de vue insecticide de la protéine selon l'une quelconque des revendications 13 à 18 dans des cellules végétales transformées, afin de lutter contre : Heliothis virescens, Helicoverpa zea, Helicoverpa armigera, Anticarsia gemmatalis, Ostrinia nubilalis, Chilo suppressalis, Chilo partellus, Scirpophaga incertulas, Sesamia inferens, Cnaphalocrocis medinalis, Marasmia patnalis, Marasmia exigua, Marasmia ruralis ou Scirpophaga innotata.

35. Utilisation de la protéine selon l'une quelconque des revendications 13 à 18 ou d'une protéine insecticide comprenant la séquence d'acides aminés de SEQ ID NO : 2 ou 4, ou comprenant la séquence d'acides aminés de SEQ ID NO : 2 de la position d'acide aminé 50 à la position d'acide aminé 625, avec une autre protéine de lutte contre les insectes qui ne reconnaît pas au moins un site de liaison reconnu par une protéine Cry2Ae.

36. Plante transgénique comprenant un ADN codant pour la protéine selon l'une quelconque des revendications 13 à 18, ou une protéine insecticide comprenant la séquence d'acides aminés de SEQ ID NO : 2 ou 3, ou comprenant la séquence d'acides aminés de SEQ ID NO : 2 de la position d'acide aminé 50 à la position d'acide aminé 625, qui a également été transformée par un ADN codant pour une protéine conférant une résistance à un herbicide à base de glufosinate ou de glyphosate.

## Revendications (Revendications pour l'(les) Etat(s) contractant(s) suivant(s): TR)

1. Séquence d'acide nucléique codant pour une protéine insecticide Cry2Ae comprenant la séquence d'acides aminés de la protéine de SEQ ID NO : 2 ou d'une partie insecticide de celle-ci, ou pour une protéine insecticide ayant au moins 99 % d'identité de Séquence avec celle-ci, ladite partie insecticide étant la plus petite partie de SEQ ID NO : 2 qui conserve une activité insecticide et qui peut être obtenue par digestion à la trypsine ou à la chymotrypsine.

2. Séquence d'acide nucléique selon la revendication 1, codant pour une protéine insecticide Cry2Ae comprenant la séquence d'acides aminés de la protéine de SEQ ID NO : 2 d'une position d'acide aminé comprise entre les positions d'acides aminés 1 et 51 à une position d'acide aminé comprise entre les positions d'acides aminés 624 et 632 de SEQ ID NO : 2.

3. Séquence d'acide nucléique selon la revendication 1, codant pour une protéine insecticide Cry2Ae comprenant la séquence d'acides aminés d'une position d'acide aminé comprise entre les positions d'acides aminés 1 et 51 à la position d'acide aminé 632 de SEQ ID NO : 2.

4. Séquence d'acide nucléique selon la revendication 1, codant pour une protéine insecticide Cry2Ae comprenant la séquence d'acides aminés de la position d'acide aminé 1 à une position d'acide aminé comprise entre les positions d'acides aminés 624 et 632 de SEQ ID NO : 2.

5. Séquence d'acide nucléique selon l'une quelconque des revendications 1 à 4, ladite séquence d'acide nucléique étant de l'ADN.

6. Séquence d'acide nucléique selon la revendication 5, comprenant une séquence d'ADN artificielle présentant un usage des codons différent par rapport à la séquence d'ADN naturellement présente, mais codant pour la même protéine ou sa partie insecticide.

7. Séquence d'acide nucléique selon la revendication 6, qui comprend la séquence nucléotidique de SEQ ID NO : 3.

8. Séquence d'acide nucléique selon la revendication 6, qui comprend la séquence nucléotidique de SEQ ID NO : 5.

9. Acide nucléique selon l'une quelconque des revendications 1 à 8, la protéine codée commençant par un dipeptide Met-Asp ou Met-Ala pour une initiation de la traduction optimale, par l'insertion, en aval du codon d'initiation, d'un codon codant pour un acide aminé Asp ou Ala en tant que nouveau deuxième codon.

10. Protéine comprenant la séquence d'acides aminés de la protéine de SEQ ID NO : 2 ou d'une partie insecticide de celle-ci, ou une protéine ayant au moins 99 % d'identité de séquence avec celle-ci, ladite partie insecticide étant la plus petite partie de SEQ ID NO : 2 qui conserve une activité insecticide et qui peut être obtenue par digestion à la trypsine ou à la chymotrypsine.

11. Protéine selon la revendication 10, comprenant la séquence d'acides aminés de SEQ ID NO : 2 d'une position d'acide aminé comprise entre les positions d'acides aminés 1 et 51 à une position d'acide aminé comprise entre les positions d'acides aminés 624 et 632 de SEQ ID NO : 2.

12. Protéine selon la revendication 10, comprenant la séquence d'acides aminés de SEQ ID NO : 2 d'une position d'acide aminé comprise entre les positions d'acides aminés 1 et 51 à la position d'acide aminé 632 de SEQ ID NO : 2.

13. Protéine selon la revendication 10, comprenant la séquence d'acides aminés de SEQ ID NO : 2 de la position d'acide aminé 1 à une position d'acide aminé comprise entre les positions d'acides aminés 624 et 632 de SEQ ID NO : 2.

14. Protéine selon l'une quelconque des revendications 10 à 13, comprenant la séquence d'acides aminés de SEQ ID NO : 4.

15. Protéine selon l'une quelconque des revendications 10 à 13, ladite protéine commençant par un dipeptide Met-Asp ou Met-Ala pour une initiation de la traduction optimale, par l'insertion, en aval du codon d'initiation, d'un codon codant pour un acide aminé Asp ou Ala en tant que nouveau deuxième codon.

16. Protéine selon l'une quelconque des revendications 10 à 15, qui inclut un peptide de transit pour l'adressage au chloroplaste.

17. Gène chimère comprenant la séquence d'acide nucléique selon l'une quelconque des revendications 1 à 9, sous le contrôle d'un promoteur exprimable chez les plantes.

18. Gène chimère selon la revendication 17, qui comprend en outre un ADN codant pour un peptide d'adressage ou de transit pour l'adressage à la vacuole, à la mitochondrie, au chloroplaste, au plaste, ou pour la sécrétion.

19. Gène chimère selon la revendication 18, ledit ADN supplémentaire codant pour un peptide de transit vers le chloroplaste.

20. Gène chimère comprenant les éléments suivants liés de manière opérationnelle :
a) un promoteur 35S issu du virus de la mosaïque du chou-fleur ;
b) une séquence de tête provenant du gène de la protéine de liaison à la chlorophylle a/b de pétunia ;
c) la séquence codante d'ADN de SEQ ID NO : 3 ; et
d) une région 3' de terminaison de la transcription et de polyadénylation du gène 35S du virus de la mosaïque du chou-fleur.

21. Gène chimère selon la revendication 20, ledit gène chimère comprenant également un ADN codant pour le peptide de transit TpssuAt permettant l'adressage au chloroplaste, inséré à l'extrémité 5' de la séquence codante *cry2Ae.*

22. Cellules végétales, plantes ou graines transformées de manière à comprendre le gène chimère selon l'une quelconque des revendications 17 à 21.

23. Cellules végétales, plantes ou graines selon la revendication 22, qui sont choisies dans le groupe constitué : du maïs, du coton, du riz, du tabac, du colza, d'espèces de Brassica, de l'aubergine, du soja, de la pomme de terre, du tournesol, de la tomate, de la canne à sucre, du thé, des haricots, de la fraise, du trèfle, du concombre, de la pastèque, du poivron, de l'avoine, de l'orge, du blé, du dahlia, du glaïeul, du chrysanthème, de la betterave sucrière, du sorgho, de la luzerne et de l'arachide.

24. Microorganisme transformé de manière à comprendre la séquence d'acide nucléique selon l'une quelconque des revendications 1 à 9.

25. Procédé permettant de rendre une plante résistante à un insecte, comprenant la transformation de cellules végétales par le gène chimère selon l'une quelconque des revendications 17 à 21 et la régénération de plantes transformées qui sont résistantes aux insectes à partir de telles cellules.

26. Procédé selon la revendication 25, ledit insecte étant choisi dans le groupe constitué : d'Heliothis virescens, d'Helicoverpa zea, d'Helicoverpa armigera, d'Anticarsia gemmatalis, d'Ostrinia nubilalis, de Chilo suppressalis, de Chilo partellus, de Scirpophaga incertulas, de Sesamia inferens, de Cnaphalocrocis medinalis, de Marasmia patnalis, de Marasmia exigua, de Marasmia ruralis et de Scirpophaga innotata.

27. Utilisation de la protéine selon l'une quelconque des revendications 10 à 16, afin de lutter contre des insectes choisis dans le groupe constitué : d'Heliothis virescens, d'Helicoverpa zea, d'Helicoverpa armigera, d'Anticarsia gemmatalis, d'Ostrinia nubilalis, de Chilo suppressalis, de Chilo partellus, de Scirpophaga incertulas, de Sesamia inferens, de Cnaphalocrocis medinalis, de Marasmia patnalis, de Marasmia exigua, de Marasmia ruralis et de Scirpophaga innotata.

28. Plante, cellule végétale ou graine comprenant le gène chimère selon l'une quelconque des revendications 17 à 19 et un deuxième gène chimère comprenant un ADN codant pour une protéine insecticide choisie parmi : une protéine Cry1F ou un fragment toxique de celle-ci ; une protéine hybride dérivée d'une protéine Cry1F, une protéine hybride Cry1A-Cry1F ; une protéine de type Cry1A ou un fragment toxique de celle-ci, une protéine Cry1Ac ou une protéine hybride Cry1Ab-Cry1Ac ; une protéine VIP3Aa ou un fragment toxique de celle-ci, ou des protéines insecticides de souches des espèces de Xhenorhabdus, Serratia ou Photorhabdus.

29. Plante de coton transgénique comprenant la protéine selon l'une quelconque des revendications 10 à 13, 15 ou 16 à titre de première protéine de lutte contre les insectes et la protéine Cry1Ac ou VIP3Aa à titre de deuxième protéine de lutte contre les insectes.

30. Utilisation de la protéine selon l'une quelconque des revendications 10 à 16 avec une autre protéine de lutte contre les insectes qui ne reconnaît pas au moins un site de liaison reconnu par une protéine Cry2Ae de ce type.

31. Plante transgénique comprenant un ADN codant pour la protéine selon l'une quelconque des revendications 10 à 13, 15 ou 16, qui a également été transformée par un ADN codant pour une protéine conférant une résistance à un herbicide à base de glufosinate ou de glyphosate.
